# EUROPÄISCHE PATENTSCHRIFT

(11) **EP 0 783 541 B1**
(45) Veröffentlichungstag und Bekanntmachung des Hinweises auf die Patenterteilung: **13.01.1999**
(21) Anmeldenummer: 95935877.1
(22) Anmeldetag: 28.09.1995
(51) Int. Cl.: C08G 73/06, C09K 11/06, H05B 33/14

(54) **STICKSTOFFHALTIGE POLYMERE ALS ELEKTROLUMINESZENZMATERIALIEN**
NITROGENOUS POLYMERS USED AS ELECTROLUMINESCENT MATERIALS
POLYMERES RENFERMANT DE L'AZOTE UTILISES COMME MATERIAUX ELECTROLUMINESCENTS

(30) Priorität: 30.09.1994 DE 4435047; 17.02.1995 DE 19505416
(43) Veröffentlichungstag der Anmeldung: 16.07.1997
(73) Patentinhaber: HOECHST AKTIENGESELLSCHAFT, 65929 Frankfurt am Main (DE)
(72) Erfinder: KREUDER, Willi, D-55126 Mainz (DE); HÖRHOLD, Hans-Heinrich, D-07747 Jena (DE); ROST, Henning, D-07743 Jena (DE)
(86) Internationale Anmeldenummer: EP9503836
(87) Internationale Veröffentlichungsnummer: WO9610598

(56) Entgegenhaltungen:
- EP-A- 0 295 084
- WO-A-90/13148
- US-A- 3 418 261
- US-A- 5 227 252
- CHEMICAL ABSTRACTS, vol. 112, no. 18, 30.April 1990 Columbus, Ohio, US; abstract no. 159825a, SILING ET AL. 'Poly(oxyphenylisoindazenes) as luminescent additives for coating and organic glass' Seite 64; & SU,A,1 521 741 (INSTITUTE OF HETEROORGANIC COMPOUNDS)

## Beschreibung

Es besteht ein hoher industrieller Bedarf an großflächigen Festkörper-Lichtquellen für eine Reihe von Anwendungen, überwiegend im Bereich von Anzeigeelementen, der Bildschirmtechnologie und der Beleuchtungstechnik. Die an diese Lichtquellen gestellten Anforderungen können zur Zeit von keiner der bestehenden Technologien völlig befriedigend gelöst werden.

Als Alternative zu herkömmlichen Anzeige- und Beleuchtungselementen, wie Glühlampen, Gasentladungslampen und nicht selbstleuchtenden Flüssigkristallanzeigeelementen, sind bereits seit einiger Zeit Elektrolumineszenz(EL)materialien und -vorrichtungen, wie lichtemittierende Dioden (LED), in Gebrauch.

In der DE-A 25 45 784 (entspricht US-A 3,995,299) ist eine Elektrolumineszenzvorrichtung mit einer Strahlungsquelle beschrieben, die aus einer Schicht eines amorphen oder überwiegend amorphen Polymermaterials mit merklicher elektrischer Ladungsbeweglichkeit und niedrigem lonisationspotential, einem starken Elektronendonor, einem starken Elektronenakzeptor und vorzugsweise zumindest einem lumineszierenden Additiv besteht, wobei elektrische Anschlüsse vorgesehen sind, durch die ein elektrischer Strom durch die Dicke der Schicht zur Anregung von Strahlung daraus geleitet werden kann.

Als Polymermaterialien sind konjugierte Polymere, wie Poly(p-phenylenvinylen) (siehe z.B. WO-A 90/13148), wie auch nicht konjugierte Polymere eingesetzt worden (siehe z.B. I. Sokolik et al., J. Appl. Phys. 1993, 74, 3584), wobei konjugierte Materialien im allgemeinen den Vorteil einer höheren Ladungsträgerbeweglichkeit und damit besserer Effizienzen und niedrigerer Einsatzspannungen haben.

Neben Vorrichtungen auf Basis von Polymeren sind seit längerem auch niedermolekulare organische Elektrolumineszenzvorrichtungen bekannt. Saito et al. (Appl. Phys. Lett. 1990, 56, 799) beschreiben solche Vorrichtungen mit Triarylaminstilbenen als lichtemittierenden Schichten.

Aus US-A-5,227,252 sind Elektrolumineszenzvorrichtungen enthaltend Chinacridinone bekannt.

Obwohl mit diesen Materialien gute Ergebnisse erzielt wurden, ist das Eigenschaftsprofil dieser Verbindungen noch durchaus verbesserungsfähig.

Da zudem die Entwicklung von Elektrolumineszenzmaterialien, insbesondere auf Grundlage von Polymeren, noch in keiner Weise als abgeschlossen betrachtet werden kann, sind die Hersteller von Beleuchtungs- und Anzeigevorrichtungen an den unterschiedlichsten Elektrolumineszenzmaterialien für solche Vorrichtungen interessiert.

Dies liegt unter anderem auch daran, daß erst das Zusammenwirken der Elektrolumineszenzmaterialien mit den weiteren Bauteilen der Vorrichtungen Rückschlüsse auf die Qualität auch des Elektrolumineszenzmaterials zuläßt.

Aufgabe der vorliegenden Erfindung war es daher, neue Elektrolumineszenzmaterialien bereitzustellen, die bei Verwendung in Beleuchtungs- oder Anzeigevorrichtungen geeignet sind, das Eigenschaftsprofil dieser Vorrichtungen zu verbessern.

Es wurde nun überraschend gefunden, daß sich bestimmte stickstoffhaltige Polymere in besonderer Weise als Elektrolumineszenzmaterialien eignen.

Polymere mit Triaryleinheiten sind teilweise als photosensitive Komponenten für elektrophotographische Verfahren und als Sensibilisatoren für elektrisch photosensitive Farbstoffe (US-A 4,323,203) bekannt, eine Eignung der strukturell unterschiedlichen Polymere der vorliegenden Erfindung als Elektrolumineszenzmaterialien läßt sich daraus jedoch nicht ableiten.

Gegenstand der Erfindung sind daher stickstoffhaltige Polymere, enthaltend Struktureinheiten der Formel (I), 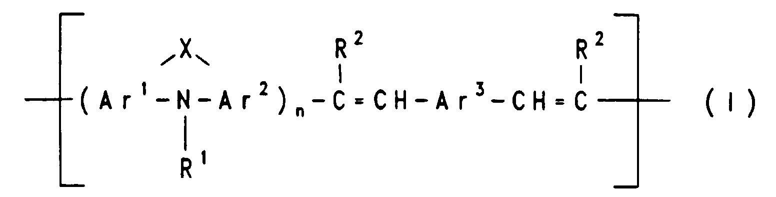 wobei die Symbole und Indizes folgende Bedeutungen haben:
- Ar¹, Ar², Ar³: sind gleich oder verschieden ein- und/oder mehrkernige, gegebenenfalls über eine oder mehrere, vorzugsweise eine Brücke verknüpfte, und/oder kondensierte Aryl- und/oder Heteroarylgruppen, vorzugsweise mit 4 bis 400, besonders bevorzugt 4 bis 100, ganz besonders bevorzugt 4 bis 20 Kohlenstoffatomen, die gegebenenfalls substituiert sein können;
- X: ist eine Einfachbindung, -O-, -S-, -SO-, -SO₂, NR³, -CR⁴R⁵-, -CO-, -CR⁶=CR⁷, CR⁸R⁹-CR¹⁰R¹¹ oder SiR¹²R¹³;
- R¹-R¹³: sind gleich oder verschieden H, ein Kohlenwasserstoffrest der gegebenenfalls substituiert sein kann und auch Heteroatome, vorzugsweise O und/oder F enthalten kann, mit 1 bis 22 Kohlenstoffatomen oder Ar⁴, wobei Ar⁴ gleich oder verschieden von Ar¹ die gleichen Bedeutungen wie Ar¹ hat;
- n: ist 1, 2 oder 3, vorzugsweise 1 oder 2, besonders bevorzugt 1.

Bevorzugt sind Polymere, bestehend aus Struktureinheiten der Formel (I). Die erfindungsgemäßen Polymere zeichnen sich unter anderem durch eine niedrige Einsatzspannung der Elektrolumineszenz und eine hohe Effizienz aus. Sie sind zudem besonders als Lochleitermaterialien geeignet.

Als Elektrolumineszenzmaterialien im Sinne der Erfindung gelten Materialien, die als aktive Schicht in einer Elektrolumineszenzvorrichtung Verwendung finden können. Aktive Schicht bedeutet, daß die Schicht befähigt ist, bei Anlegen eines elektrischen Feldes Licht abzustrahlen (lichtemittierende Schicht) und/oder daß sie die Injektion und/oder den Transport der positiven und/oder negativen Ladungen verbessert (Ladungsinjektions- oder Ladungstransportschicht).

Gegenstand der Erfindung sind daher auch die Verwendung eines Polymers, enthaltend Struktureinheiten der Formel (I), als Elektrolumineszenzmaterial, insbesondere als Lochleitermaterial, und Elektrolumineszenzmaterialien, enthaltend ein oder mehrere Polymere, die Struktureinheiten der Formel (I) enthalten.

Die erfindungsgemäßen Polymere weisen im allgemeinen 2 bis 1000, vorzugsweise 3 bis 500, besonders bevorzugt 4 bis 300, Wiederholeinheiten auf.

Bevorzugt sind Polymere, enthaltend Struktureinheiten der Formel (I), bei denen die Symbole und Indizes folgende Bedeutungen haben:
- Ar¹, Ar²: sind gleich oder verschieden, vorzugsweise gleich, 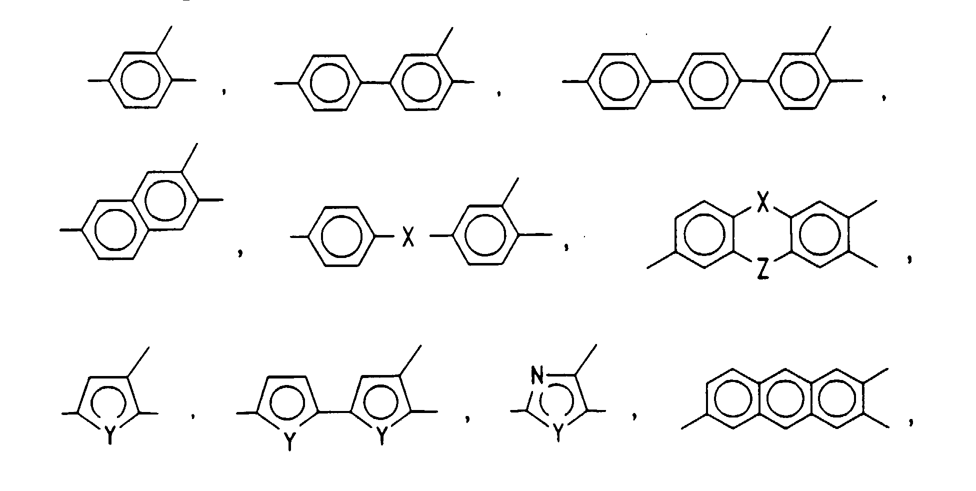 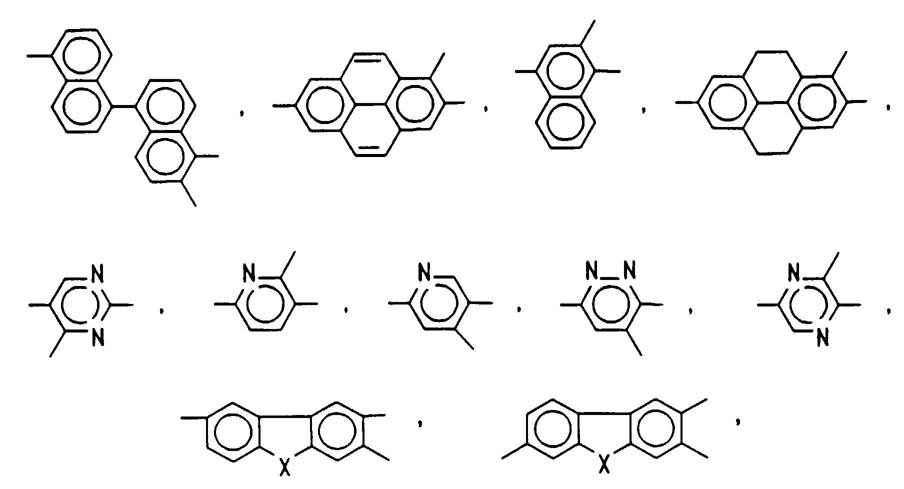
- Ar³: hat, gleich oder verschieden von Ar¹, Ar³, die gleichen Bedeutungen wie Ar¹, wobei von den drei möglichen Bindungsstellen jeweils nur zwei realisiert sind oder ist 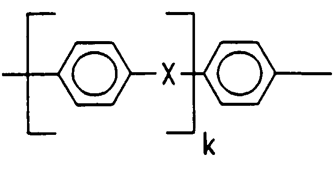 mit k = 1 bis 20, vorzugsweise 1, 2 oder 3;
- Ar¹, Ar², Ar³: können dabei gleich oder verschieden mit einem oder mehreren Resten R¹⁴ substituiert sein;
- X, Z: sind gleich oder verschieden, eine Einfachbindung, -O-, -S-, -SO-, -SO₂-, -NR³, -CR⁴R⁵-, -CO-, -CR⁶=CR⁷-, -CR⁸R⁹-CR¹⁰R¹¹ oder SiR¹²R¹³;
- Y:: -O-, -S- oder -NR³-;
- R¹-R¹³: ist H, eine geradkettige oder verzweigte Alkylgruppe mit 1 bis 22 Kohlenstoffatomen, wobei auch eine oder zwei nicht benachbarte CH₂-Gruppen durch -O-, -S-, -CO-, -CO-O-, -O-OC- oder -Si(CH₃)₂- ersetzt sein können, eine Cycloalkylgruppe mit 3 bis 10 Kohlenstoffatomen oder Ar⁴, wobei Ar⁴ gleich oder verschieden von Ar¹⁻³ die gleichen Bedeutungen wie Ar¹⁻³ hat, jedoch von den drei möglichen Bindungen nur eine realisiert ist, R⁴-R¹¹ sind auch F, CF₃, O-CF₃ oder CN;
- R¹⁴: ist gleich oder verschieden H, eine Alkylgruppe mit 1 bis 22, vorzugsweise 1 bis 12, Kohlenstoffatomen, wobei auch eine oder zwei nicht benachbarte CH₂-Gruppen durch -O-, -S-, -CO-, -CO-O-, -O-OC- oder -Si(CH₃)₂- ersetzt sein können, -CF₃, -Ph, -O-Ph, -S-Ph, -SO-Ph, -SO₂-Ph, F, Cl, Br, I oder -CN;
- n: 1, 2 oder 3, vorzugsweise 1 oder 2, besonders bevorzugt 1.

Besonders bevorzugt sind Polymere, enthaltend Struktureinheiten der Formel (I), bei denen die Symbole und Indizes folgende Bedeutungen haben:

Die Gruppe 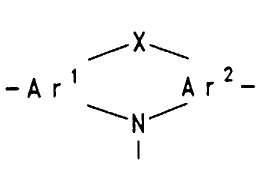 ist 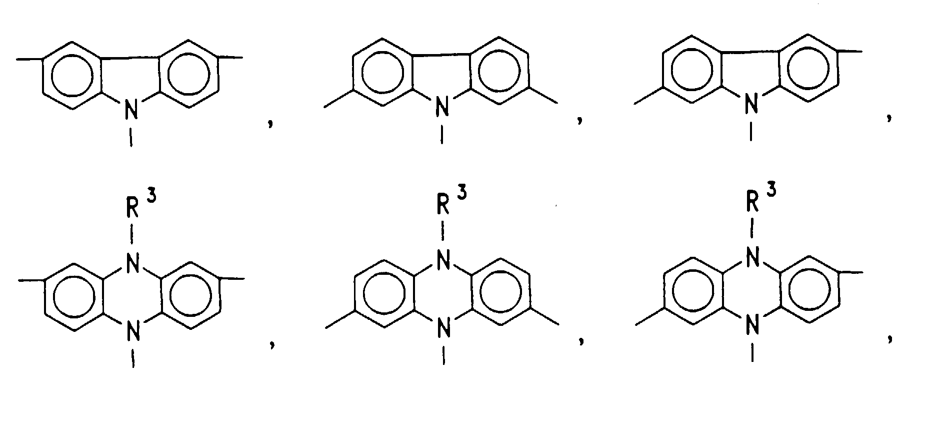 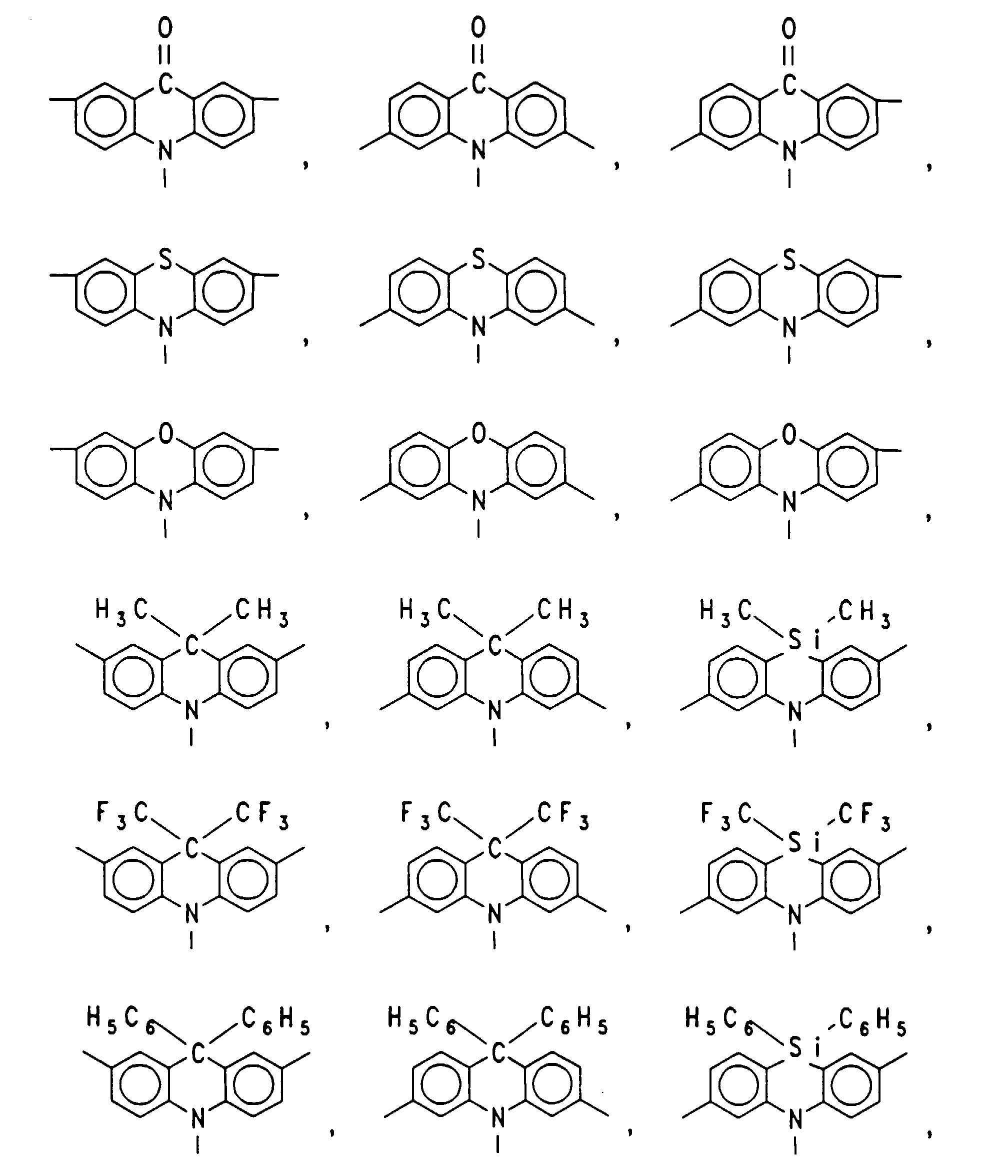
- Ar³: ist 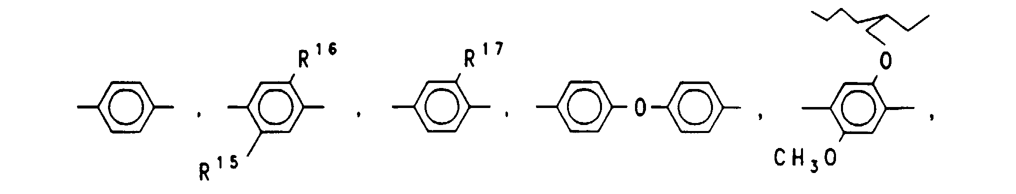 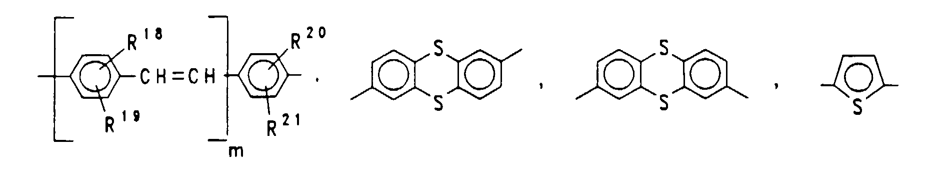 mit m = 1 ... 20, vorzugsweise 1, 2 oder 3, besonders bevorzugt 1, 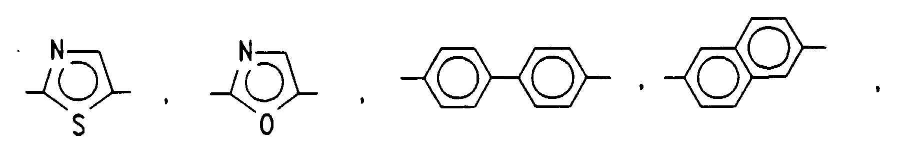
- R¹, R²: haben gleich oder verschieden von Ar³ die gleichen Bedeutungen wie Ar³, wobei jedoch nur eine der beiden möglichen Bindungsstellen zum Polymer realisiert ist, oder sind 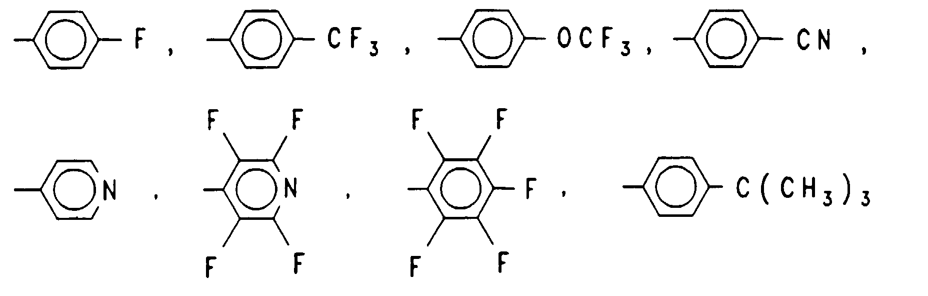 oder H, eine geradkettige oder verzweigte Alkylgruppe mit 1 bis 12 Kohlenstoffatomen, wobei auch eine oder zwei nicht benachbarte CH₂-Gruppen durch -O-, -S-, -CO-, -CO-O-, -O-OC- oder -Si(CH₃)₂- ersetzt sein können, eine Cycloalkylgruppe mit 3 bis 6 Kohlenstoffatomen oder eine Aralkylgruppe mit 7 bis 11 Kohlenstoffatomen;
- R¹⁵-R²¹: sind gleich oder verschieden F, Cl, eine geradkettige oder, verzweigte Alkyl- oder Alkoxygruppe mit 1 bis 22, vorzugsweise 1 bis 12, Kohlenstoffatomen,
- R¹⁸-R²¹: können auch Wasserstoff sein.

Ganz besonders bevorzugt sind Polymere, enthaltend Struktureinheiten der Formel (I), bei denen Ar¹ und Ar² die gleiche Bedeutung haben, sowie Polymere bei denen R² H oder Ar⁴ ist.

Bei den erfindungsgemäßen Polymeren kann es sich um Homo- oder Copolymere handeln. Copolymere enthalten unterschiedliche Struktureinheiten der Formel (I) und/oder weitere Struktureinheiten, die konjugiert oder nicht konjugiert sein können, beispielsweise 1,4-Phenylen- oder α,ω-Alkylen-Gruppen.

Die Herstellung der erfindungsgemäßen oder erfindungsgemäß verwendeten Polymere erfolgt zweckmäßigerweise durch Kondensation von Bis-aldehyden bzw. Bisketonen der Formel (II), 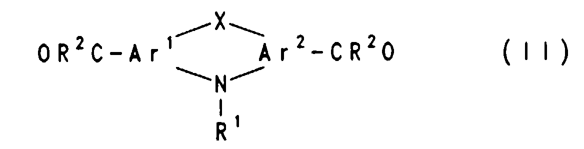 wobei Ar¹, Ar², R¹, R², und X die in der Formel (I) angegebenen Bedeutungen haben, mit phosphororganischen Verbindungen der Formel (III) 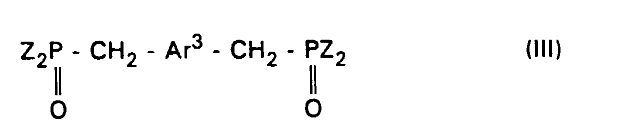 wobei Ar³ die in der Formel (I) angegebenen Bedeutungen besitzt und Z für C₁-C₂₂-Alkoxy, vorzugsweise Ethoxy, oder Arylreste, vorzugsweise Phenyl, steht.

Die Kondensation erfolgt durch Einwirkung eines basischen Kondensationsmittels, vorzugsweise von Kalium-tert.-butylat oder Natriumhydrid.

Die Polykondensation wird zweckmäßigerweise so vorgenommen, daß man das äquimolare Gemisch der Ausgangskomponenten (II) und (III) in einem Lösungsmittel vorlegt und unter Inertgasatmosphäre und Rühren vorzugsweise mindestens molare Mengen Kondensationsmittel in Lösung oder Suspension einträgt.

Nach einer anderen Arbeitsvariante kann das Kondensationsmittel auch allein oder mit dem Bisketon in einem Lösungsmittel vorgelegt und die Bisphosphorkomponente zugegeben werden. Als Lösungsmittel werden vorzugsweise Benzol, Toluol, Xylol oder Dimethylformamid verwendet,die Reaktionstemperatur beträgt vorzugsweise 60 bis 120°C und die Reaktionszeit 0,1 bis 20, vorzugsweise 0,1 bis 5, besonders bevorzugt 0,1 bis 1 Stunden. Die Umsetzungen erfolgen nahezu quantitativ.

Durch Zusatz von Wasser, gegebenenfalls einer Säure, wie Essigsäure, und Abtrennung der organischen Reaktionsphasen kann die Aufarbeitung erfolgen. Die erhaltenen Kondensationsprodukte können zur Reinigung extrahiert, z.B. mit Alkoholen oder Essigsäure, oder aus Lösung in einem Lösungsmittel mittels eines Nichtlösungsmittels gefällt werden.

Allgemein ist dieses Herstellungsverfahren beispielsweise in DD 84 272, Hörhold, H.-H.: Z. Chem. 12, 41-52 (1972); Hörhold, H.-H.; Bergmann, R.; Gottschaldt, J.; Drefahl, G.: Acta Chim. Acad. Sci. Hung. 81, 239-251; Hörhold, H.-H.; Bergmann, R.: Advances in the Chemistry of Thermally Stable Polymers, Warszawa, Polish Scientific Publishers, 29-48 (1977); Hörhold, H.-H.; Helbig, M.: Makromol. Chem., Macromol. Symp. 12, 229-258 (1987) und Hörhold, H.-H.; Helbig, M.; Raabe, D.; Opfermann, J.; Scherf, U., Stockmann, R.; Weiß, D.: Z. Chem. 27,126 (1987) beschrieben.

Die bei der Horner-Reaktion entstehenden E/Z-lsomere, sowie Isomere, die sich aus der möglichen Stellung der beiden Doppelbindungen zueinander ergeben (trans-trans-anti, trans-trans-syn, cis-trans-anti, cis-trans-syn, cis-cis-anti, cis-cis-syn); werden alle von der Erfindung umfaßt.

Durch den Einsatz unterschiedlicher Bisaldehyde bzw. Bisketone und/oder Bisphosphonate werden in einfacher Weise Copolymere erhalten, die unterschiedliche Struktureinheiten der Formel (I) aufweisen. In solchen Copolymeren können die Reste R¹ in der Formel (I) gegebenenfalls auch unterschiedliche Bedeutungen haben. Weiterhin ist durch verzögerte Zugabe von mindestens einem der Comonomere die Möglichkeit gegeben, Blockcopolymere herzustellen.

Kleinere Gruppen von Polymeren enthaltend Struktureinheiten der Formel (I), bei denen Ar¹ die Bedeutung Ar⁵-Ar⁵ hat, wobei Ar⁵ für einen elektronenreichen Aromaten, vorzugsweise Thiophen-2,5-ylen, das gegebenenfalls auch substituiert sein kann, steht, können alternativ auch oxidativ, z.B. mit FeCl₃ (siehe u.a. P. Kovacic, N. B. Jones, Chem. Ber. 1987, 87, 357 bis 379; M. Weda, T. Abe, H. Awano, Macromolecules 1992, 25, 5125), oder elektrochemisch (siehe z.B. N. Saito, T. Kanbara, T. Sato, T. Yamamoto, Polym. Bull. 1993, 30, 285) polymerisiert werden:

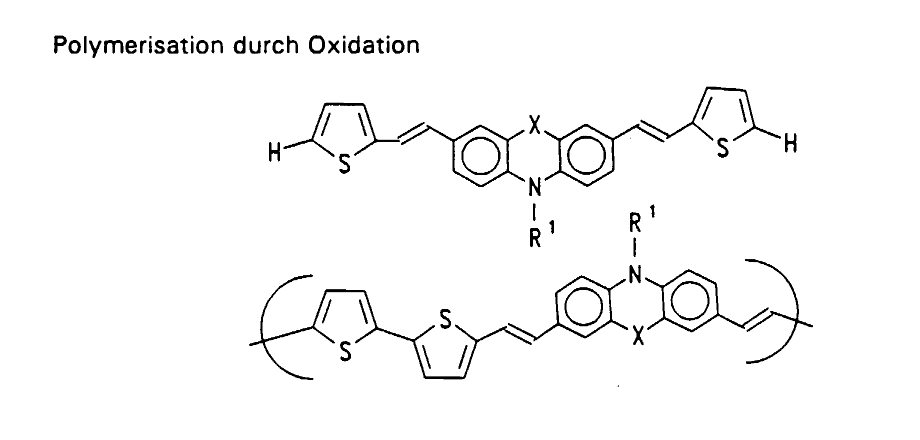

Ebenso ist es möglich, aromatische Verbindungen unter den Bedingungen der Scholl-Reaktion oxidativ zu kuppeln: 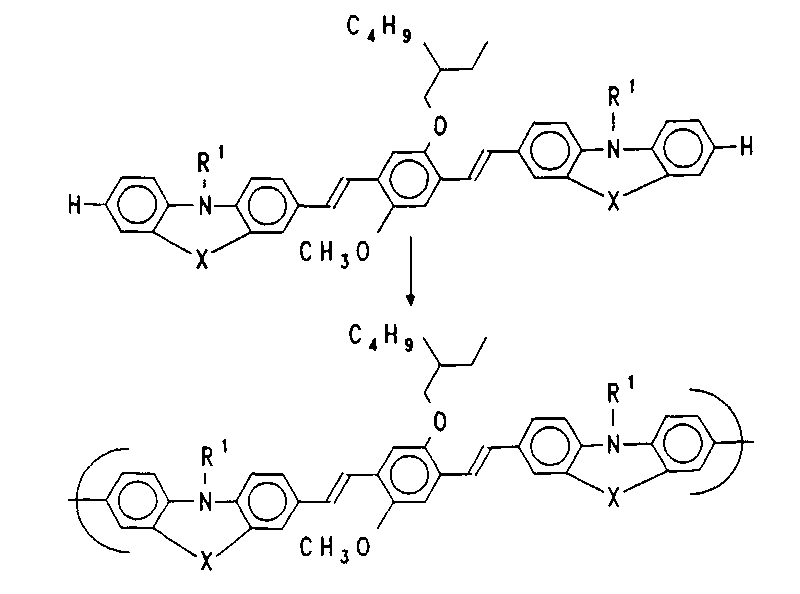 (siehe z.B. J. March, Advanced Organic Chemistry, 3. Aufl. S. 484 f., McGraw Hill und die dort zitierte Literatur).

Es kann zweckmäßig sein, zur Molmassenregulierung bei der Polymerisation monofunktionelle Aldehyde oder Ketone zur Bildung definierter Endgruppen zuzusetzen, beispielsweise den kommerziell erhältlichen N-Ethyl-3-carbazolcarboxy-aldehyd (Fa. Aldrich, Milwaukee, USA) oder die folgende, aus 4-Fluorbenzaldehyd leicht zugängliche Verbindung.

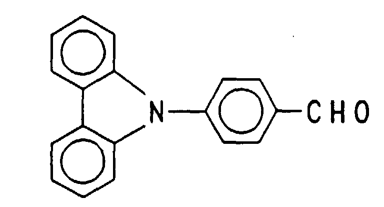

Werden zwei Mol eines monofunktionellen Aldehyds oder Ketons mit einem Mol einer phosphororganischen Verbindung der Formel (III) eingesetzt, erhält man Sesquimere der Formel (la), 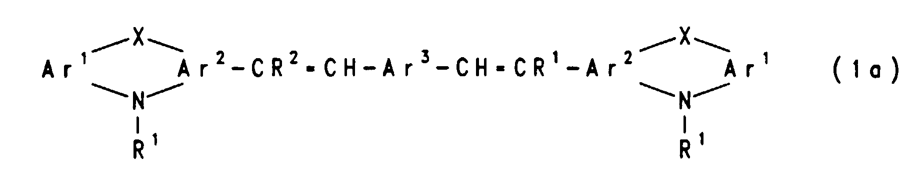 wobei die Symbole und Indizes die gleichen Bedeutungen und Bevorzugungen wie in der Formel (I) haben.

Solche Verbindungen eignen sich, insbesondere in Mischung mit den erfindungsgemäßen Polymeren, ebenfalls als Elektrolumineszenzmaterialien.

Bei der Polymerisation von Ausgangsverbindungen mit meta-substituierten Strukturelementen können sich makrocyclische Verbindungen bilden.

Die erfindungsgemäßen oder erfindungsgemäß verwendeten Polymere können daher auch in Mischung mit diesen makrocyclischen Verbindungen vorliegen, was den Einsatz als Elektrolumineszenzmaterial nicht stört.

Die Herstellung der Ausgangsverbindungen (II) und (III) erfolgt nach an sich literaturbekannten Methoden, wie sie in Standardwerken zur Organischen Synthese, z.B. Houben-Weyl, Methoden der Organischen Chemie, Georg-Thieme-Verlag, Stuttgart, beschrieben werden.

Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch gemacht werden.

Die als Kondensationskomponenten benötigten Bis(diphenylphosphanoxide) bzw. Bis(phosphonsäureester) sind beispielsweise unter Anwendung der Michaelis-Arbusov-Reaktion aus den entsprechenden Bis(halogenmethyl)-Verbindungen mit Diphenylphosphinigsäureethylester (C₆H₅)₂P-O-C₂H₅ oder mit Triethylphosphit leicht zugänglich.

Die Synthese von Bisaldehyden der Formel (II) kann nach verschiedenen, dem Fachmann geläufigen Reaktionstypen erfolgen.

So können Aldehyde beispielsweise aus Bis-carbonsäurederivaten durch kontrollierte Reduktion mit Reduktionsmitteln wie Lithium-tris-alkoxyalanaten oder H₂/Pd ("Rosenmund-Reduktion") erhalten werden: 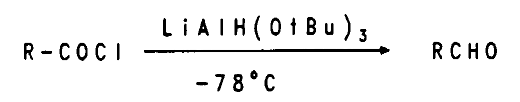 (siehe z.B. Fuson in Patai, "The Chemistry of the Carbonyl Group", Vol. 1, S. 211-232, Interscience, New York 1966) 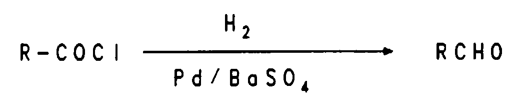 (siehe z.B. Tylander, "Catalytic Hydrogenation over Platinum Metals, S. 398-404, Academic Press, New York 1967).

Aus Bischlormethyl-Vorstufen lassen sich beispielsweise durch die Sommelet-Reaktion Bisaldehyde erhalten 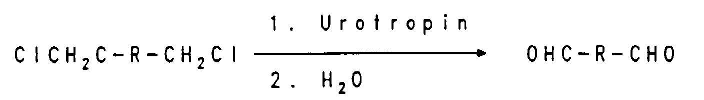 (siehe z.B. Angyal, Organic Reactions 1954, 8, 197)

Bevorzugt werden jedoch die zugrundeliegende Amine (IV) und (V) 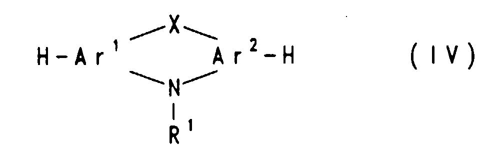 einer elektrophilen aromatischen Substitution unterworfen.
Dafür sind zahlreiche Methoden bekannt, beispielsweise die Gattermann-Koch-Reaktion: 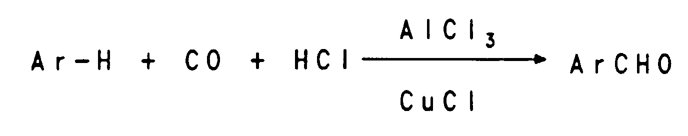 (siehe z.B. Crounse, Organic Reactions 1949, 5, 290-300);
die Gattermann Reaktion: 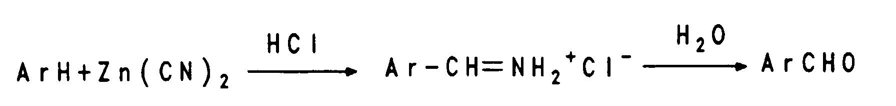 (siehe z.B. Truce, Organ. Reactions 1957, 9, 37-72);
oder die Reaktion von Aromaten mit Dichlormethylmethylether: 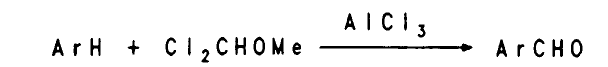 (siehe z.B. Rieche et al., Chem. Ber. 1960, 93, 88 oder Lewin et al., Org. Prep. Proced. Int. 1978, 10, 201).

Bevorzugt ist jedoch die Vilsmeier Reaktion (auch mit DMF): 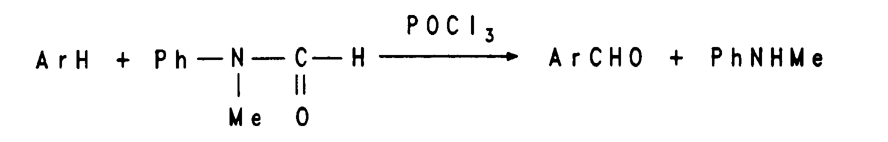 (siehe z.B. Jutz in Advances in Organic Chemistry,Vol. 9, Teil 1 S. 225-342, Böhme, Viche Eds. Interscience, New York 1976, oder Jackson, J. Am. Chem. Soc. 1981, 103, 533).

Falls R¹ in der Formel (I) nicht Wasserstoff ist, werden Bisketone als Ausgangsstoffe verwendet.

Diese können beispielsweise durch die oben beschriebene Vilsmeier-Reaktion hergestellt werden, wenn das eingesetzte Amid kein Formamid ist.

Weiterhin können Bisketone durch die Friedel-Crafts-Acylierung hergestellt werden: 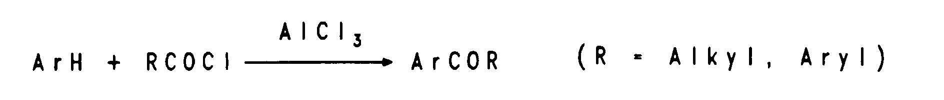 (siehe beispielsweise Olah, Friedel-Crafts and Related Reactions, vol. 2, 979-1047, Interscience, New York 1963-65);
oder durch eine Grignard-Reaktion mit Cyanoarylen: 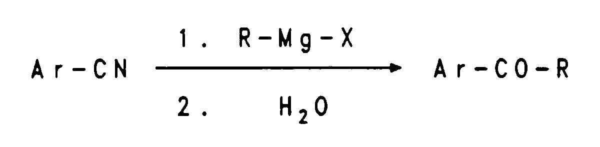 (siehe z.B. Kharasch u. Rainmuth, Grignard Reactions of Nonmetallic Substances, S. 767-845, Prentice Hall, Eaglewood Cliffs N.J. 1954). Ausgangsverbindungen für Bisaldehyde und Bisketone sind die Amine (IV). Sie lassen sich nach bekannten, dem Fachmann geläufigen Methoden herstellen.

Solche Methoden sind beispielsweise beschrieben in A.R. Katritzky und 1. M. Lagowski, The Prinicples of Heterocyclic Chemistry, Methuen Vlg., London 1967 oder in den entsprechenden Bänden der Serie "The chemistry of Heterocyclic Compounds" von A. Weissberger und E.C. Taylor (Herausgeber).

So können die Amine der Formel (IV) beispielsweise aus den entsprechenden Diiodverbindungen erhalten werden:

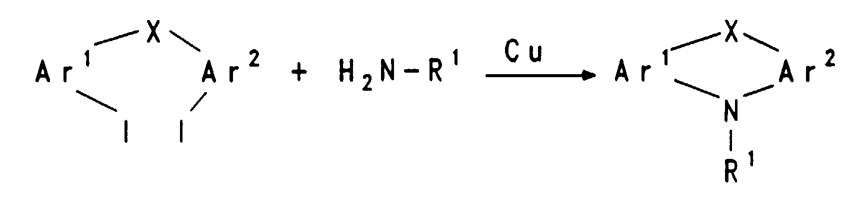

Viele der Verbindungen sind auch kommerziell erhältlich, beispielsweise: Carbazol, N-Methylcarbazol, N-Phenylcarbazol, N-Biphenylcarbazol, Dihydrophenazin, Acridinon, Phenoxazin (Dibenzoxazin), Phenothiazin (Dibenzothiazin), alle Fa. Aldrich, Steinheim, Deutschland oder 9,9-Diphenyl-9,10-dihydro-9-sila-acridin und 9,9-Dibenzyl-9,10-dihydro-9, sila-acridin (Fa. Salor, Steinheim, Deutschland).

Um als Elektrolumineszenzmaterialien Verwendung zu finden, werden die erfindungsgemäßen Polymere im allgemeinen nach bekannten, dem Fachmann geläufigen Methoden, wie Eintauchen (Dipping) oder Spincoating, in Form eines Films auf ein Substrat aufgebracht.

Gegenstand der Erfindung ist daher auch ein Verfahren zur Herstellung eines Elektrolumineszenzmaterials, dadurch gekennzeichnet, daß man
a) eine phosphororganische Verbindung der Formel (III) 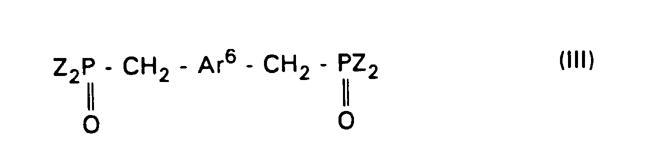 mit einem Bisaldehyd oder Bisketon der Formel (II) 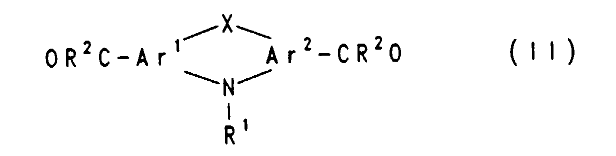 unter Einwirkung eines basischen Kondensationsmittels zu einem Polymer, enthaltend Struktureinheiten der Formel (I), kondensiert, 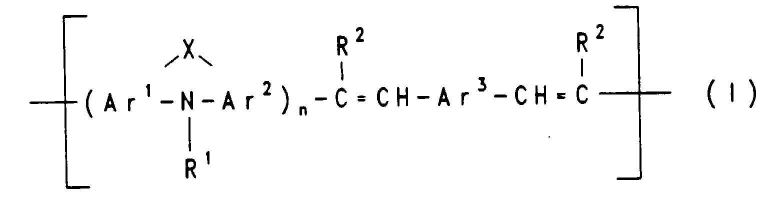 wobei die Symbole und Indizes folgende Bedeutungen haben:
   - Ar¹, Ar², Ar³: sind gleich oder verschieden ein- und/oder mehrkernige, gegebenenfalls über eine oder mehrere, vorzugsweise eine Brücke verknüpfte, und/oder kondensierte Aryl- und/oder Heteroarylgruppen, vorzugsweise mit 4 bis 400, besonders bevorzugt 4 bis 100, ganz besonders bevorzugt 4 bis 20 Kohlenstoffatomen, die gegebenenfalls substituiert sein können;
   - X: ist eine Einfachbindung, -O-, -S-, -SO-, -SO₂, NR³, -CR⁴R⁵-, -CO-, -CR⁶ = CR⁷, CR⁸R⁹-CR¹⁰R¹¹ oder SiR¹²R¹³;
   - R¹-R¹³: sind gleich oder verschieden H, ein Kohlenwasserstoffrest mit 1 bis 22 Kohlenstoffatomen oder Ar⁴, wobei Ar⁴ die gleichen Bedeutungen wie Ar¹ haben kann;
   - n: ist 1, 2 oder 3, vorzugsweise 1 oder 2, besonders bevorzugt 1,
   und
b) das so erhaltene Polymer, enthaltend Struktureinheiten der Formel (I), in Form eines Films auf ein Substrat, das gegebenenfalls bereits andere Schichten enthält, aufbringt.

Gegenstand der Erfindung ist weiterhin eine Elektrolumineszenzvorrichtung mit einer oder mehreren aktiven Schichten, wobei mindestens eine dieser aktiven Schichten ein oder mehrere erfindungsgemäße Polymere enthält. Die aktive Schicht kann beispielsweise eine lichtemittierende Schicht und/oder eine Transportschicht und/oder eine Ladungsinjektionsschicht sein. Vorzugsweise ist sie eine lichtemittierende- oder eine Lochleiterschicht.

Der allgemeine Aufbau solcher Elektrolumineszenzvorrichtungen ist beispielsweise in US 4,539,507 und US 5,151,629 beschrieben. Polymere enthaltende Elektrolumineszenzvorrichtungen sind beispielsweise in WO-A 90/13148 oder EP-A 0 443 861 beschrieben.

Sie enthalten üblicherweise eine elektrolumineszierende Schicht zwischen einer Kathode und einer Anode, wobei mindestens eine der Elektroden transparent ist. Zusätzlich kann zwischen der elektrolumineszierenden Schicht und der Kathode eine Elektroneninjektions- und/oder Elektronentransportschicht eingebracht sein und/oder zwischen der elektrolumineszierenden Schicht und der Anode eine Lochinjektions- und/oder Lochtransportschicht eingebracht sein. Als Kathode können Metalle und Metall-Legierungen, z.B. Ca, Mg, Al, In, Mg/Ag dienen. Als Anode können Metalle, z.B. Au oder andere metallisch leitende Stoffe, z.B. ITO (Indiumoxid/Zinnoxid) auf einem transparentem Substrat, z.B. aus Glas oder einem transparenten Polymer, dienen.

Im Betrieb wird die Kathode auf negatives Potential gegenüber der Anode gesetzt. Dabei werden Elektronen von der Kathode in die Elektroneninjektionsschicht-/Elektronentransportschicht bzw. direkt in die lichtemittierende Schicht injiziert. Gleichzeitig werden Löcher von der Anode in die Lochinjektionsschicht/Lochtransportschicht bzw. direkt in die lichtemittierende Schicht injiziert.

Die injizierten Ladungsträger bewegen sich unter dem Einfluß der angelegten Spannung durch die aktiven Schichten aufeinander zu. Dies führt an der Grenzfläche zwischen Ladungstransportschicht und lichtemittierender Schicht bzw. innerhalb der lichtemittierenden Schicht zu Elektronen/Loch-Paaren, die unter Aussendung von Licht rekombinieren.
Die Farbe des emittierten Lichtes kann durch die als lichtemittierende Schicht verwendeten Materialien variiert werden.

Elektrolumineszenzvorrichtungen finden Anwendung z.B. als selbstleuchtende Anzeigeelemente, wie Kontrollampen, alphanumerische Displays, Hinweisschilder, und in optoelektronischen Kopplern.

Die Erfindung wird durch die Beispiele näher erläutert, ohne sie dadurch beschränken zu wollen.

Es bedeuten
- Tg:: Glasübergangstemperatur, gemessen mittels Differential-Scanning-Calorimetry (DSC)
- Mₙ:: Zahlenmittel des Molekulargewichts
- VPO:: Vapor Pressure Osmometry (siehe z.B. Cherdron, Kern, Braun, Praktikum der Makromolekularen Chemie)
- GPC:: Gelpermeationschromatographie, Standard Polystyrol

### Beispiele

### Beispiel 1

### 3,6-Dibenzoyl-9-methylcarbazol

3,6-Dibenzoyl-9-methylcarbazol (4): 67,0 g (0,37 mol) 9-Methylcarbazol wurde in 400 ml trockenem Dichlorethan gelöst und 105,0 g (0,79 mol) AlCl₃ zugegeben. Unter Rühren und Eiskühlung wurden 104,0 g (0,74 mol) Benzoylchlorid zugetropft. Bei Raumtemperatur wurde noch 3 Stunden gerührt und danach das Reaktionsgemisch mit Eis und Salzsäure zersetzt und die organische Phase mit Wasser und NaHCO₃-Lösung neutralgewaschen. Restliches Wasser wurde durch azeotrope Destillation entfernt. Nach Einengen der Dichlorethanlösung kristallisierte das Diketon aus. Es wurde noch einmal aus Dichlorethan umkristallisiert; Schmp. 222°C; (Lit. ¹⁶⁾: Schmp. 219°C), farblose Kristalle. Ausb.: 114,2 g (79,3 %).

| | | | |
|---|---|---|---|
| C₂₇H₁₉NO₂ (389,5) | Ber. C 83,27 | H 4,92 | N 3,60 |
| | Gef. C 83,45 | H 4,86 | N 3,50 |

### Beispiel 2

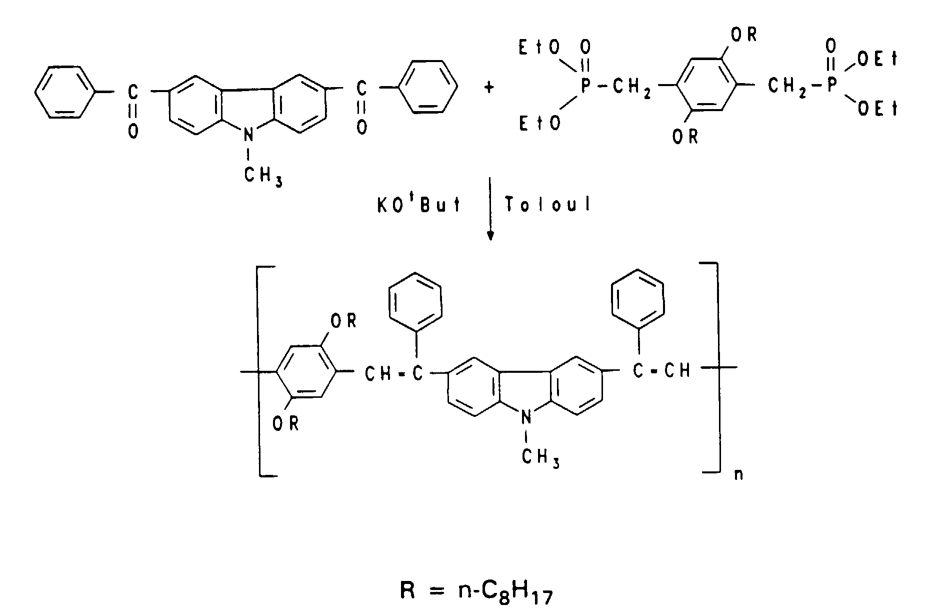

Poly(9-methylcarbazol-3,6-diyl-1-phenyl-1,2-ethylen-2,5-dioctoxy-1,4-phenylen-2-phenyl-1,2-ethylen)

| | |
|---|---|
| 3,6-Dibenzol-9-methylcarbazol | [389,1] = 1,83 g (0,0047 mol) |
| 2,5-Dioctoxy-p-xylen-bis(diethylphosphat) | [634,7] = 3,00 g (0,0047 mol) |
| Kalium-tert.-butylat Lösungsmittel:Toluol | [112,2] = 2,00 g (0,0178 mol) |

Das Diketon und das Bisphosphonat werden unter Rühren und Schutzgas in wenig Toluol in der Siedehitze gelöst und mit festem Kalium-tert.-butylat in einer Portion versetzt. Die Mischung siedet heftig auf und wird schlagartig viskos. Nach 10 Minuten versetzt man mit ca. 20 ml Toluol, um eine bessere Rührbarkeit zu gewährleisten. Man rührt noch zwei Stunden, zieht dann das Lösungsmittel ab, nimmt in Chloroform auf und fällt in Isopropanol. Die erhaltenen gelben Flocken werden 5 Stunden mit Methanol extrahiert und noch einmal umgefällt (Chloroform/Methanol). Nach dem Trocknen erhält man ein gelbes Pulver in 68 %iger Ausbeute.
analytische Daten:
- Glastemperatur Tg: 90°C
- Fluoreszenz λ_{max. em} = 505 nm θ = 30 %
- UV/VIS = λ_{max. abs} = 400,8 nm lg ε = 4,5
- E^{ox-1} = 0,91 V vs Ag/AgCl
- Mn (VPO) = 4500
- Mn (GPC) = 5200 Mw (GPC) = 11.300

### Beispiel 3

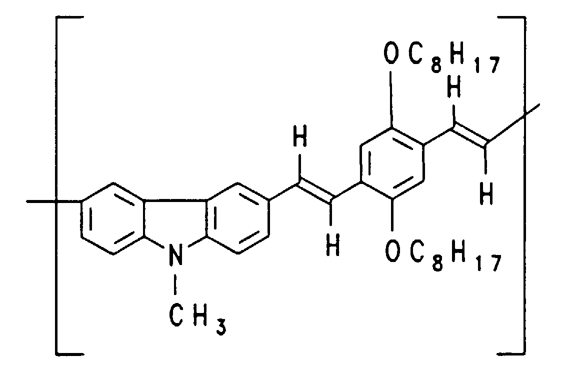

Das Ausgangsmaterial 3,6-Diformyl-N-methyl-carbazol wurde durch Vilsmeier-Reaktion erhalten. Die weitere Synthese erfolgte analog Beispiel 2.
M_{w} > 10 000
Ø_{PL} 75 %, das Polymer ist in Chlorbenzol löslich, und schwerlöslich in Chloroform.

### Beispiel 4

### Elektrolumineszenz-Vorrichtung

Eine 2 gew.-%ige Lösung des Polymers aus Beispiel 2 in Chlorbenzol wird unter Stickstoff durch Spin-Coating bei 500 upm auf einen mit ITO (Indium-Zinn-Oxid) beschichteten Glasträger (strukturiert, Streifen 2 mm breit) aufgebracht. Der Glästräger wird über eine Schleuse unter Beibehaltung der Schutzgasatmosphäre in eine Hochvakuum-Bedampfungsanlage überführt. Bei 2x10⁻⁵ mbar werden quer zu den ITO-Streifen unter Verwendung einer Maske Ca-Streifen (2 mm breit, 230 nm dick) auf die Polymerschicht aufgedampft. Die so erhaltene Vorrichtung, ITO/Polymer/Ca, wird in einen Probenhalter gegeben und die Elektroden über Federkontakte mit einer Stromquelle verbunden, wobei ein ITO-Streifen positiv und ein Ca-Streifen negativ gepolt werden. Beim Anlegen einer genügend hohen Spannung, wird an dem entsprechenden Matrixelement eine grüne Elektrolumineszenz beobachtet.
- Einsatzspannung:: 7,0 V
- Max. Effizient:: 0,032 %
Max. Leuchtdichte 120 Cd/m² bei 13 V und 7 mA/4 mm²

## Patentansprüche

1. Polymer, enthaltend Struktureinheiten der Formel (I), 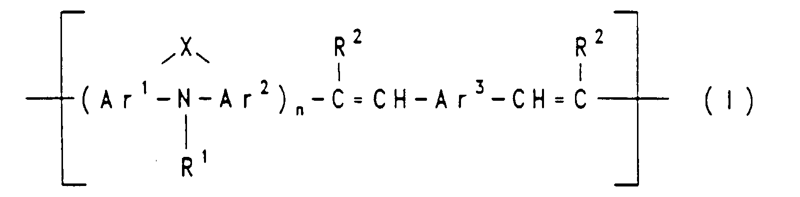 wobei die Symbole und Indizes folgende Bedeutungen haben:
Ar¹, Ar², Ar³ sind gleich oder verschieden ein- und/oder mehrkernige, gegebenenfalls über eine oder mehrere Brücken verknüpfte, und/oder kondensierte Aryl- und/oder Heteroarylgruppen, mit 4 bis 400 Kohlenstoffatomen, die gegebenenfalls substituiert sein können;
X ist eine Einfachbindung, -O-, -S-, -SO-, -SO₂, NR³, -CR⁴R⁵-, -CO-, -CR⁶=CR⁷, CR⁸R⁹-CR¹⁰R¹¹ oder SiR¹²R¹³;
R¹-R¹³ sind gleich oder verschieden H, ein Kohlenwasserstoffrest der gegebenenfalls substituiert sein kann und auch Heteroatome, vorzugsweise O und/oder F enthalten kann, mit 1 bis 22 Kohlenstoffatomen oder Ar⁴, wobei Ar⁴ gleich oder verschieden von Ar¹ die gleichen Bedeutungen wie Ar¹ hat;
n ist 1, 2 oder 3.

2. Polymer nach Anspruch 1, dadurch gekennzeichnet, daß es aus Struktureinheiten der Formel (I) besteht.

3. Polymer nach Anspruch 1 und/oder 2, dadurch gekennzeichnet, daß 2 bis 1000 Wiederholeinheiten aufweist.

4. Polymer nach einem oder mehreren der vorhergehenden Ansprüche, dadurch gekennzeichnet, daß es ein Copolymer ist.

5. Polymer nach Anspruch 4, dadurch gekennzeichnet, daß es unterschiedliche Struktureinheiten der Formel (I) aufweist.

6. Polymer nach einem oder mehreren der vorhergehenden Ansprüche, dadurch gekennzeichnet, daß die Symbole und Indizes in der Formel (I) folgende Bedeutungen haben:
Ar¹, Ar² sind gleich oder verschieden 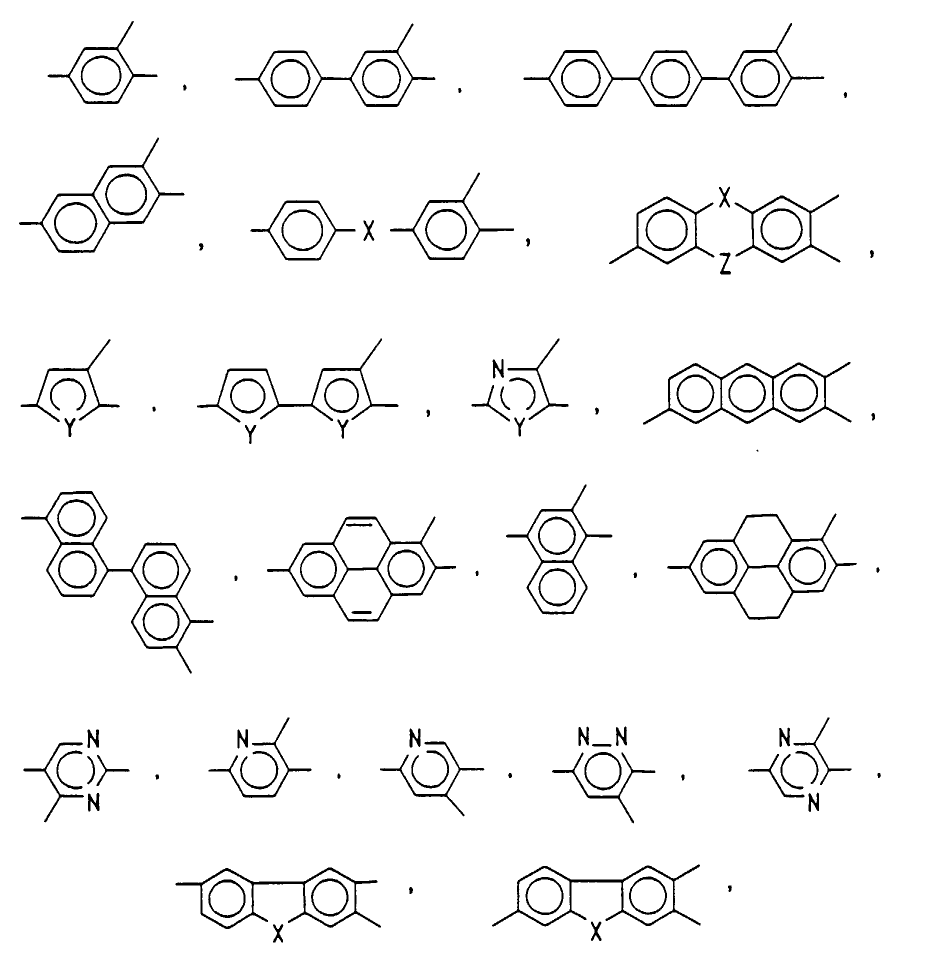
Ar³ hat, gleich oder verschieden von Ar¹, Ar³, die gleichen Bedeutungen wie Ar¹, wobei von den drei möglichen Bindungsstellen jeweils nur zwei realisiert sind oder ist 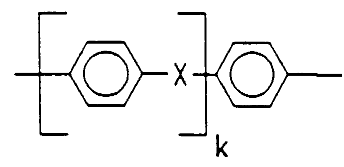 mit k = 1 bis 20;
Ar¹, Ar², Ar³ können dabei gleich oder verschieden mit einem oder mehreren Resten R¹⁴ substituiert sein;
X, Z sind gleich oder verschieden, eine Einfachbindung, -O-, -S-, -SO-, -SO₂-, -NR³, -CR⁴R⁵-, -CO-, -CR⁶ = CR⁷-, -CR⁸R⁹-CR¹⁰R¹¹ oder SiR¹²R¹³;
Y: -O-, -S- oder -NR³-;
R¹-R¹³ ist H, eine geradkettige oder verzweigte Alkylgruppe mit 1 bis 22 Kohlenstoffatomen, wobei auch eine oder zwei nicht benachbarte CH₂-Gruppen durch -O-, -S-, -CO-, -CO-O-, -O-OC- oder -Si(CH₃)₂- ersetzt sein können, eine Cycloalkylgruppe mit 3 bis 10 Kohlenstoffatomen oder Ar⁴, wobei Ar⁴ gleich oder verschieden von Ar¹⁻³ die gleichen Bedeutungen wie Ar¹⁻³ hat, jedoch von den drei möglichen Bindungen nur eine realisiert ist, R⁴-R¹¹ sind auch F, CF₃, O-CF₃ oder CN;
R¹⁴ ist gleich oder verschieden H, eine Alkylgruppe mit 1 bis 22, Kohlenstoffatomen, wobei auch eine oder zwei nicht benachbarte CH₂-Gruppen durch -O-, -S-, -CO-, -CO-O-, -O-OC- oder -Si(CH₃)₂- ersetzt sein können, -CF₃, -Ph, -O-Ph, -S-Ph, -SO-Ph, -SO₂-Ph, F, Cl, Br, I oder -CN;
n 1, 2 oder 3.

7. Polymer nach einem oder mehreren der vorhergehenden Ansprüche, dadurch gekennzeichnet, daß die Symbole und Indizes in der Formel (I) folgende Bedeutungen haben:
die Gruppe 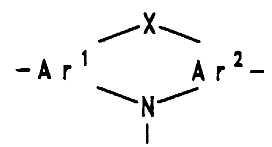 ist 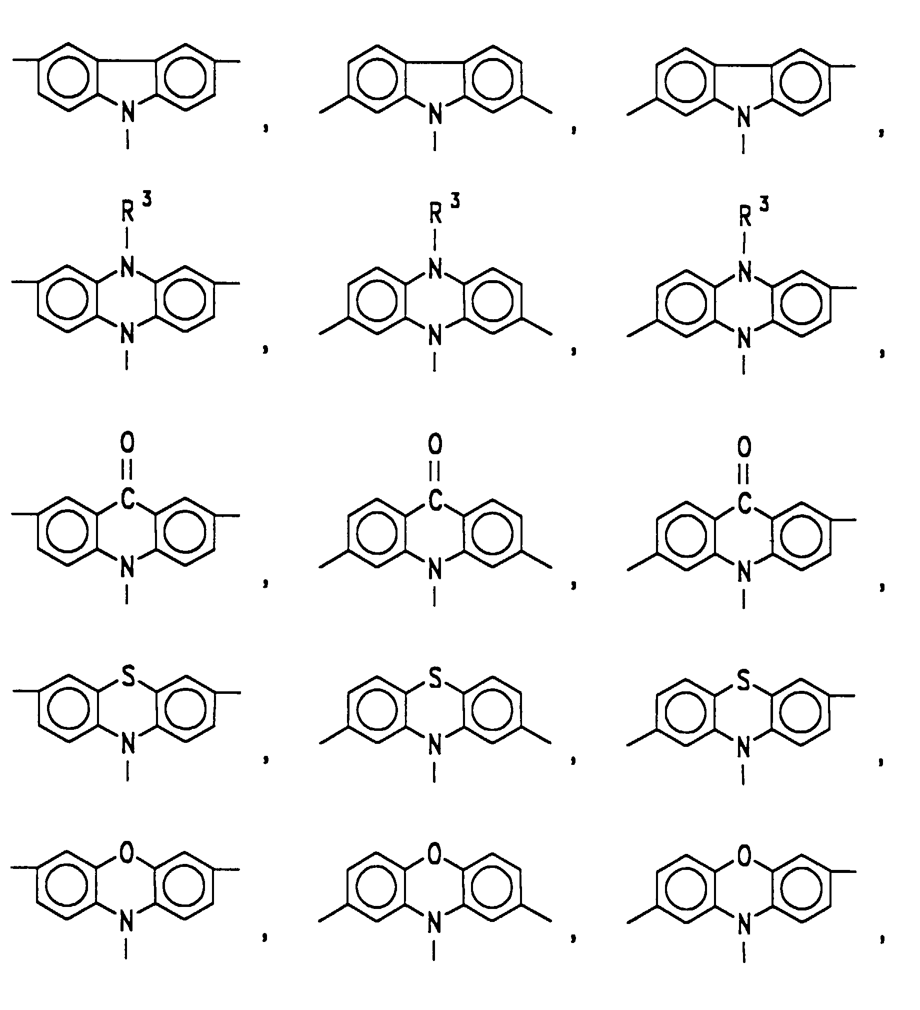 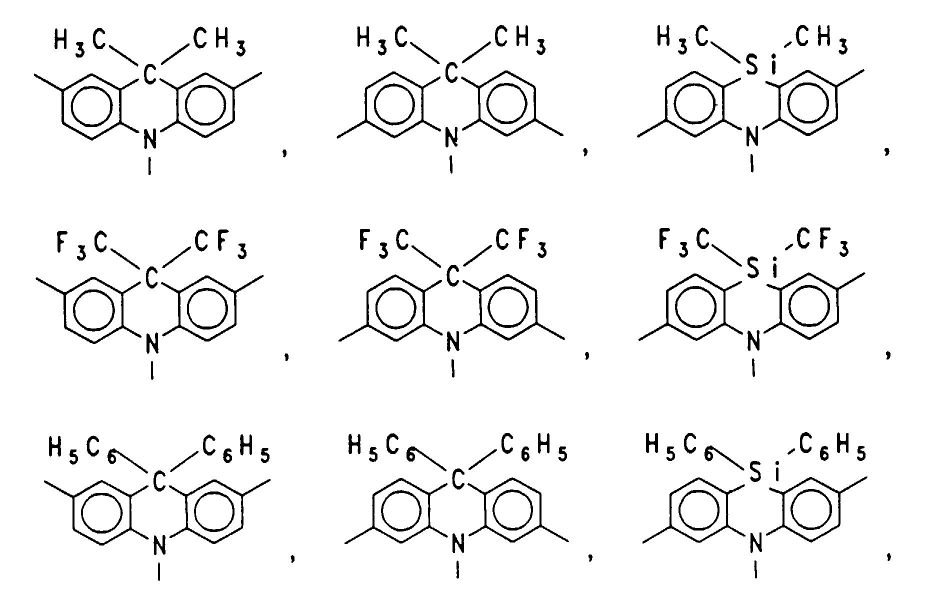
Ar³ ist 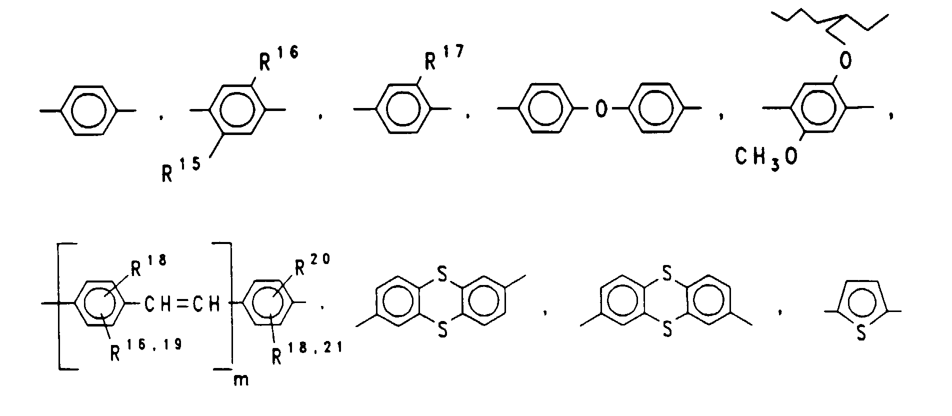 mit m = 1 ... 20. vorzuasweise 1, 2 oder 3, 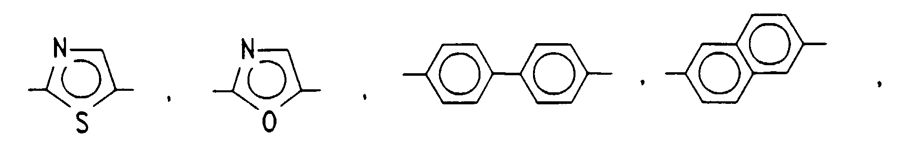
R¹, R² haben gleich oder verschieden von Ar³ die gleichen Bedeutungen wie Ar³, wobei jedoch nur eine der beiden möglichen Bindungsstellen zum Polymer realisiert ist, oder sind 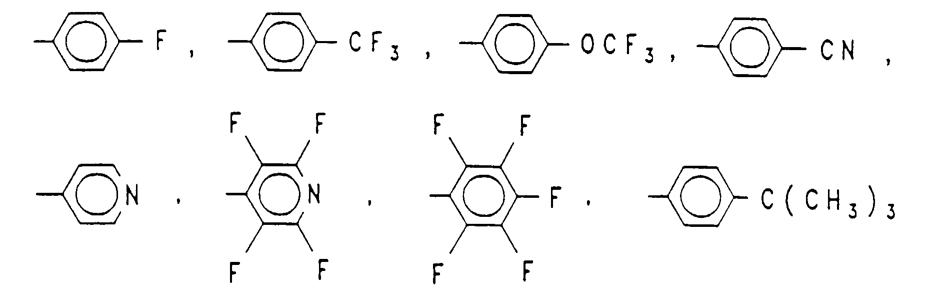 oder H, eine geradkettige oder verzweigte Alkylgruppe mit 1 bis 12 Kohlenstoffatomen, wobei auch eine oder zwei nicht benachbarte CH₂-Gruppen durch -O-, -S-, -CO-, -CO-O-, -O-OC- oder -Si(CH₃)₂- ersetzt sein können, eine Cycloalkylgruppe mit 3 bis 6 Kohlenstoffatomen oder eine Aralkylgruppe mit 7 bis 11 Kohlenstoffatomen;
R¹⁵-R²¹ sind gleich oder verschieden F, Cl, eine geradkettige oder verzweigte Alkyl- oder Alkoxygruppe mit 1 bis 22, Kohlenstoffatomen,
R¹⁸-R²¹ können auch Wasserstoff sein.

8. Polymer nach einem oder mehreren der vorhergehenden Ansprüche, dadurch gekennzeichnet, daß Ar¹ und Ar² gleich sind.

9. Verfahren zur Herstellung eines Polymers nach einem oder mehreren der vorhergehenden Ansprüchen, dadurch gekennzeichnet, daß Bis-aldehyde bzw. Bisketone der Formel (II), 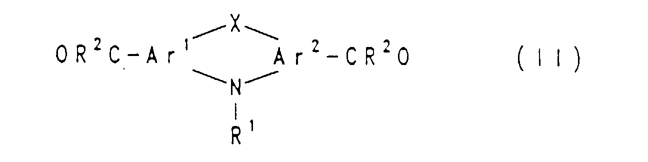 wobei Ar¹, Ar², R¹, R², und X die in der Formel (I) angegebenen Bedeutungen haben, mit phosphororganischen Verbindungen der Formel (III) 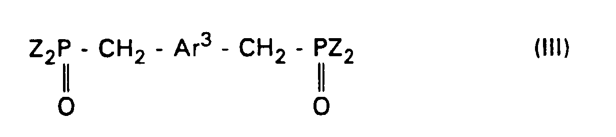 kondensiert werden, wobei Ar³ die in der Formel (I) angegebenen Bedeutungen besitzt und Z für C₁-C₂₂-Alkoxy, steht.

10. Verwendung eines Polymers nach einem oder mehreren der Ansprüche 1 bis 8 als Elektrolumineszenzmaterial.

11. Elektrolumineszenzmaterial, enthaltend ein oder mehrere Polymere nach einem oder mehreren der Ansprüche 1 bis 8.

12. Verfahren zur Herstellung eines Elektrolumineszenzmaterials nach Anspruch 11, dadurch gekennzeichnet, daß man
a) eine phosphororganische Verbindung der Formel (III) 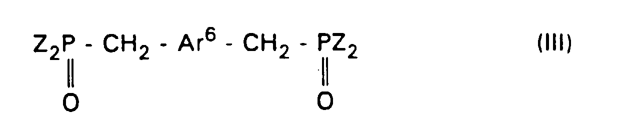 mit einem Bisaldehyd oder Bisketon der Formel (II) 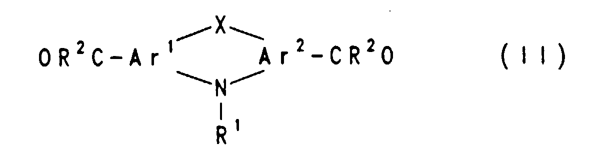 unter Einwirkung eines basischen Kondensationsmittels zu einem Polymer, enthaltend Struktureinheiten der Formel (I), kondensiert, 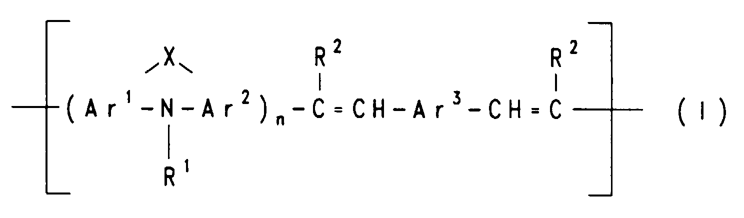 wobei die Symbole und Indizes folgende Bedeutungen haben:
Ar¹, Ar², Ar³ sind gleich oder verschieden ein- und/oder mehrkernige, gegebenenfalls über eine oder mehrere Brücken verknüpfte, und/oder kondensierte Aryl- und/oder Heteroarylgruppen, mit 4 bis 400 Kohlenstoffatomen, die gegebenenfalls substituiert sein können;
X ist eine Einfachbindung, -O-, -S-, -SO-, -SO₂, NR³, -CR⁴R⁵-, -CO-, -CR⁶=CR⁷, CR⁸R⁹-CR¹⁰R¹¹ oder SiR¹²R¹³;
R¹-R¹³ sind gleich oder verschieden H, ein Kohlenwasserstoffrest mit 1 bis 22 Kohlenstoffatomen oder Ar⁴, wobei Ar⁴ gleich oder verschieden von Ar¹ die gleichen Bedeutungen wie Ar¹ hat;
n ist 1, 2 oder 3,
und
b) das so erhaltene Polymer, enthaltend Struktureinheiten der Formel (I), in Form eines Films auf ein Substrat, das gegebenenfalls bereits andere Schichten enthält, aufbringt.

13. Elektrolumineszenzvorrichtung mit einer oder mehreren aktiven Schichten, wobei mindestens eine dieser aktiven Schichten ein oder mehrere Polymere nach einem oder mehreren der Ansprüche 1 bis 8 enthält.

## Claims

1. A polymer comprising structural units of formula (I), 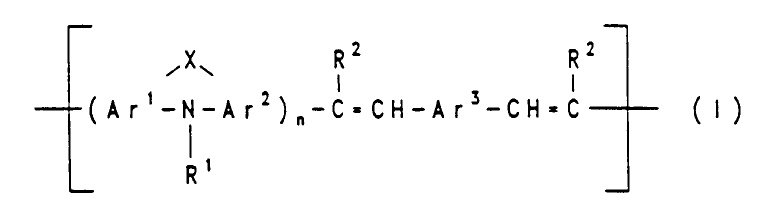 where the symbols and indices have the following meanings:
Ar¹, Ar², Ar³ are, identically or differently, mono- and/or polynuclear and/or condensed aryl and/or heteroaryl groups which may or may not be linked via one or more bridges, having from 4 to 400 carbon atoms, which may or may not be substituted;
X is a single bond, -O-, -S-, -SO-, -SO₂, NR³, -CR⁴R⁵-, -CO-, -CR⁶=CR⁷, CR⁸R⁹-CR¹⁰R¹¹ or SiR¹²R¹³;
R¹-R¹³ are, identically or differently, H, a hydrocarbon radical which may or may not be substituted and may also contain hetero atoms, preferably O and/or F, having from 1 to 22 carbon atoms or Ar⁴, where Ar⁴, identical with or different from Ar¹, has the same meanings as Ar¹;
n is 1, 2 or 3.

2. The polymer as claimed in claim 1, which consists of structural units of formula (I).

3. The polymer as claimed in claim 1 and/or 2, which comprises from 2 to 1000 repeating units.

4. The polymer as claimed in one or more of the preceding claims which is a copolymer.

5. The polymer as claimed in claim 4, which comprises different structural units of formula (I).

6. The polymer as claimed in one or more of the preceding claims, wherein the symbols and indices in formula (I) have the following meanings:
Ar¹, Ar² are, identically or differently, 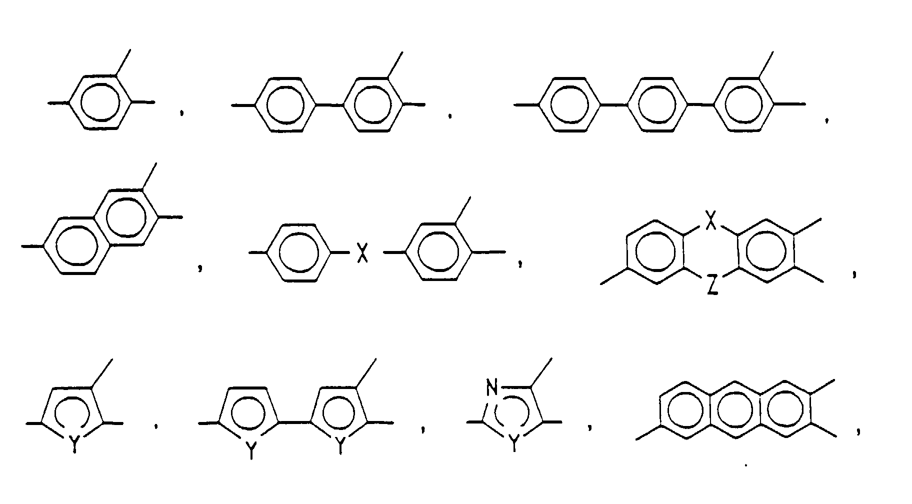 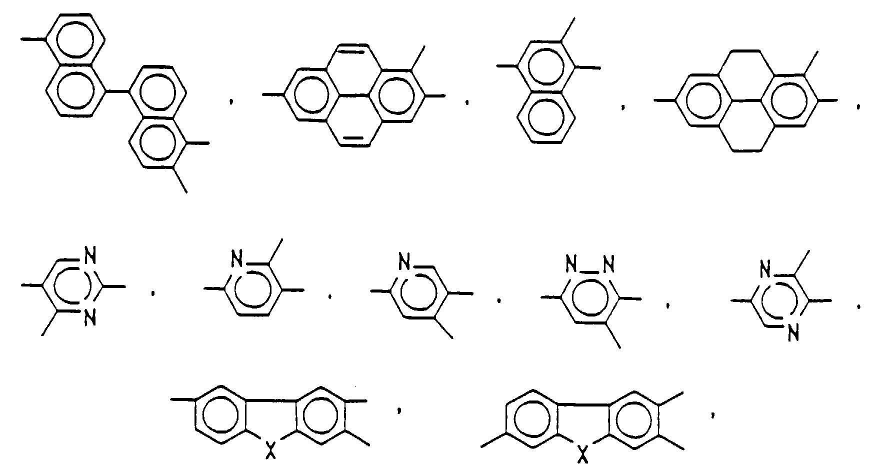
Ar³, identical with or different from Ar¹, Ar³, has the same meanings as Ar¹, only two of the three possible bonding sites being implemented in each case, or is 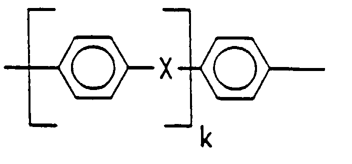 with k = 1 to 20;
Ar¹, Ar², Ar³ in this context may be substituted identically or differently with one or more radicals R¹⁴;
X, Z are, identically or differently, a single bond, -O-, -S-, -SO-, -SO₂-, -NR³, -CR⁴R⁵-, -CO-, -CR⁶=CR⁷-, -CR⁸R⁹-CR¹⁰R¹¹ or SiR¹²R¹³;
Y: -O-, -S- or -NR³-;
R¹-R¹³ is H, a straight-chain or branched alkyl group having from 1 to 22 carbon atoms, one or two nonadjacent CH₂ groups optionally being replaced by -O-, -S-, -CO-, -CO-O-, -O-OC- or -Si(CH₃)₂-, a cycloalkyl group having from 3 to 10 carbon atoms or Ar⁴, where Ar⁴, identical with or different from Ar¹⁻³, has the same meanings as Ar¹⁻³, but only one of the three possible bonds is implemented, R⁴-R¹¹ also being F, CF₃, O-CF₃ or CN;
R¹⁴ is, identically or differently, H, an alkyl group having from 1 to 22 carbon atoms, one or two nonadjacent CH₂ groups optionally being replaced by -O-, -S-, -CO-, -CO-O-, -O-OC- or -Si(CH₃)₂-, -CF₃, -Ph, -O-Ph, -S-Ph, -SO-Ph, -SO₂-Ph, F, Cl, Br, I or -CN;
n is 1, 2 or 3.

7. The polymer as claimed in one or more of the preceding claims, wherein the symbols and indices in formula (I) have the following meanings:
the group 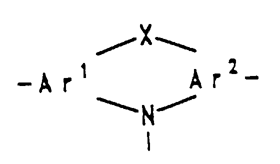 is 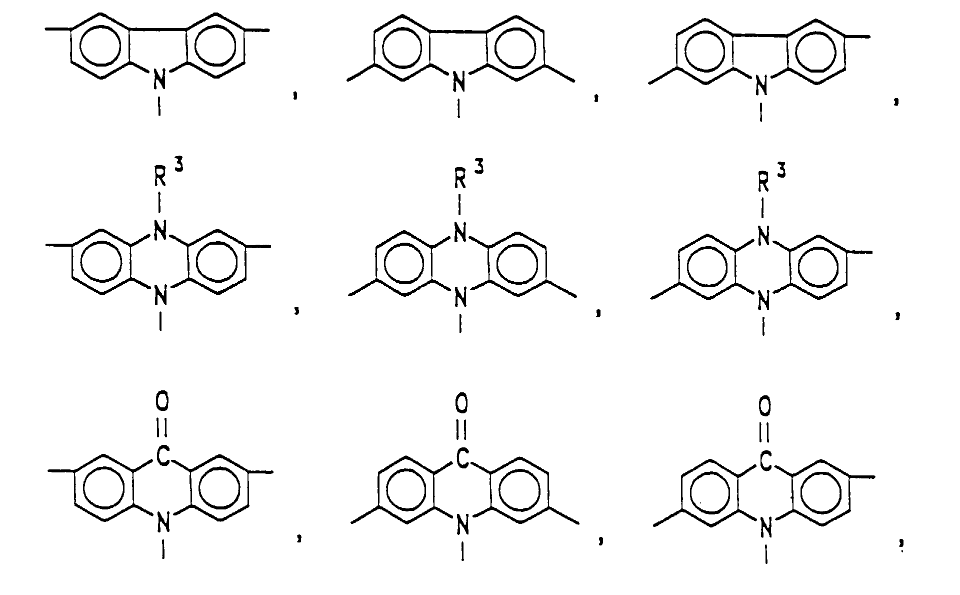 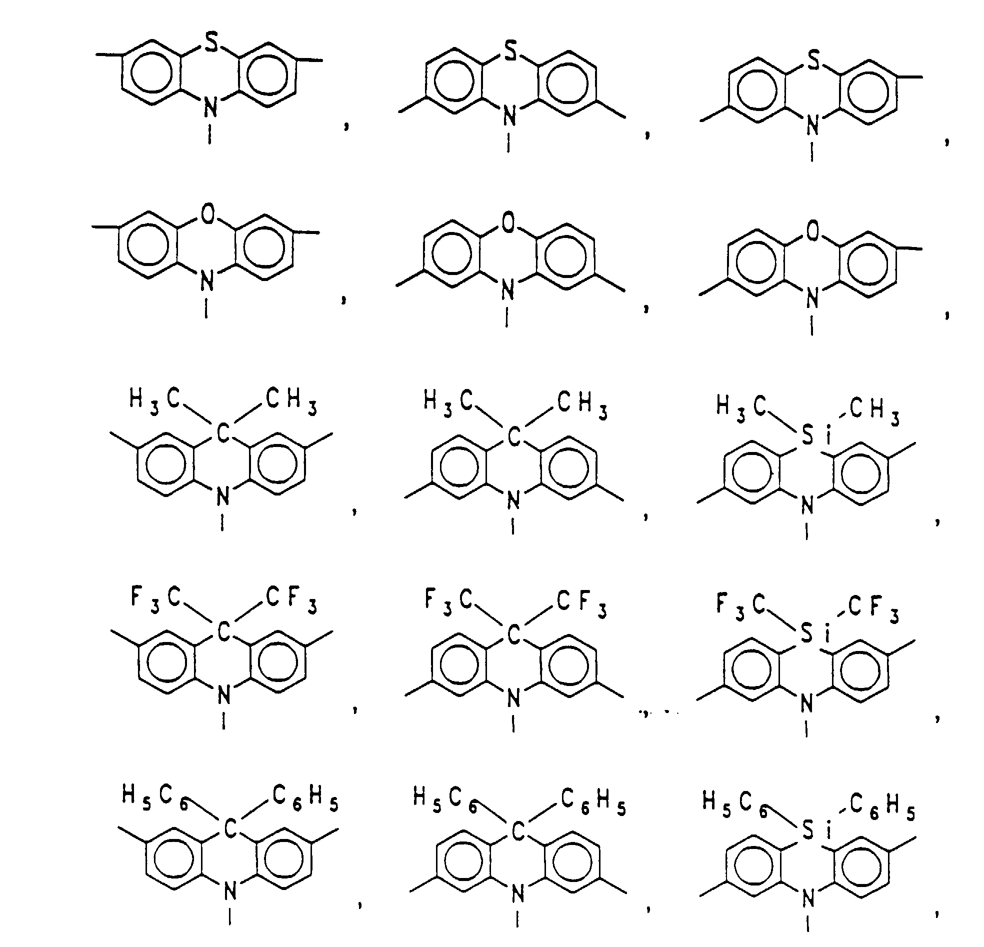
Ar³ is 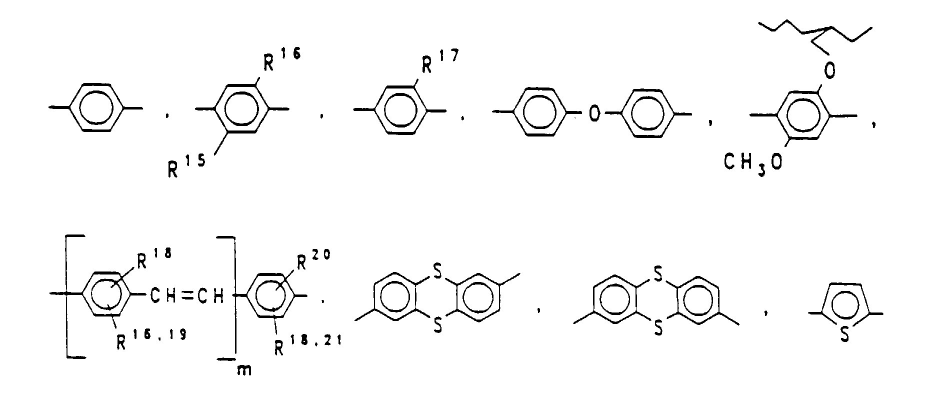 where m = 1 ... 20, preferably 1, 2 or 3, 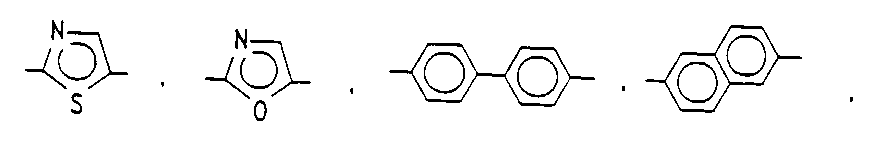
R¹, R², identical with or different from Ar³, have the same meanings as Ar³, only one of the two possible bonding sites to the polymer being implemented, however, or are 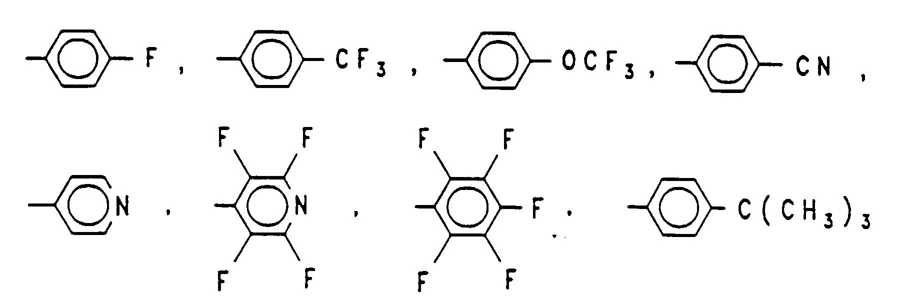 or H, a straight-chain or branched alkyl group having from 1 to 12 carbon atoms, one or two nonadjacent CH₂ groups optionally being replaced by -O-, -S-, -CO-, -CO-O-, -O-OC- or -Si(CH₃)₂-, a cycloalkyl group having from 3 to 6 carbon atoms or an aralkyl group having from 7 to 11 carbon atoms;
R¹⁵-R²¹ are, identically or differently, F, Cl, a straight-chain or branched alkyl or alkoxy group having from 1 to 22 carbon atoms,
R¹⁸-R²¹ may also be hydrogen.

8. The polymer as claimed in one or more of the preceding claims, wherein Ar¹ and Ar² are identical.

9. A method for preparing a polymer as claimed in one or more of the preceding claims, wherein bisaldehydes or bisketones of formula (II) 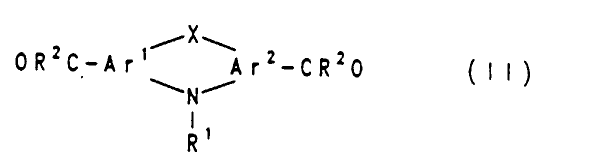 where Ar¹, Ar², R¹, R² and X have the meanings specified in formula (I) are condensed with organophosphorus compounds of formula (III) 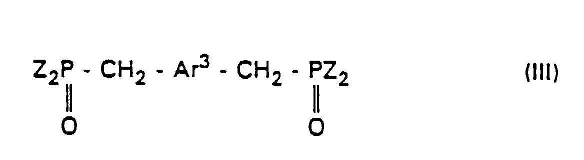 where Ar³ has the meanings specified in formula (I) and Z is C1-C22-alkoxy.

10. The use of a polymer as claimed in one or more of claims 1 to 8 as an electroluminescent material.

11. An electroluminescent material comprising one or more polymers as claimed in one or more of claims 1 to 8.

12. A method for preparing an electroluminescent material as claimed in claim 11, which comprises
a) condensing an organophosphorus compound of formula (III) 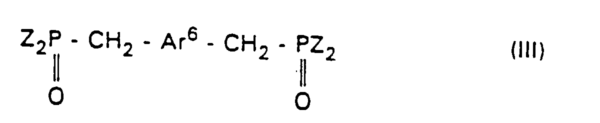 with a bisaldehyde or bisketone of formula (II) 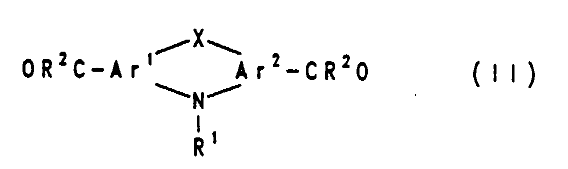 in the presence of a basic condensing agent to produce a polymer comprising structural units of formula (I), 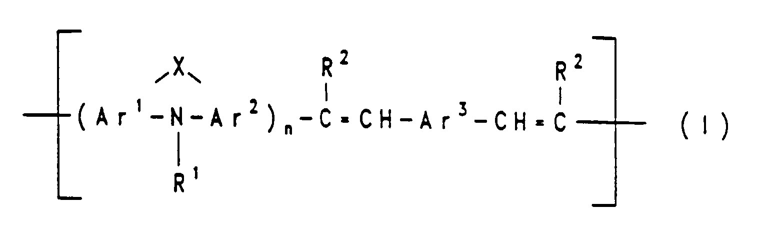 where the symbols and indices have the following meanings:
Ar¹, Ar², Ar³ are, identically or differently, mono- and/or polynuclear and/or condensed aryl and/or heteroaryl groups which may or may not be linked via one or more bridges, having from 4 to 400 carbon atoms, which may or may not be substituted;
X is a single bond, -O-, -S-, -SO-, -SO₂, NR³, -CR⁴R⁵-, -CO-, -CR⁶=CR⁷, CR⁸R⁹-CR¹⁰R¹¹ or SiR¹²R¹³;
R¹-R¹³ are, identically or differently, H, a hydrocarbon radical having from 1 to 22 carbon atoms or Ar⁴, where Ar⁴, identical with or different from Ar¹, has the same meanings as Ar¹;
n is 1, 2 or 3,
and
b) applying the resulting polymer which contains structural units of formula (I) as a film to a substrate which may or may not already contain other layers.

13. An electroluminescence device comprising one or more active layers, at least one of said active layers comprising one or more polymers as claimed in one or more of claims 1 to 8.

## Revendications

1. Polymère renfermant de l'azote, contenant des motifs de structure de formule (I) : 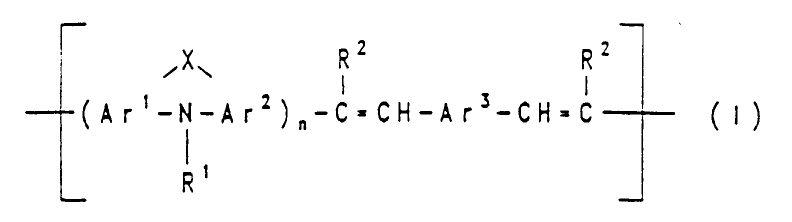 dans laquelle les symboles et les indices ont les significations suivantes :
Ar¹, Ar², Ar³ sont des groupes aryle et/ou hétéroaryle identiques ou différents, avec de 4 à 400 atomes de carbone, à un et/ou plusieurs cycles, éventuellement couplés par un ou plusieurs ponts, et/ou condensés, qui peuvent être éventuel-lement substitués;
X est une simple liaison, -O-, -S-, -SO-, -SO₂-, NR³, -CR⁴R⁵-, -CO-, -CR⁶=CR⁷, CR⁸R⁹-CR¹⁰R¹¹ ou SiR¹²R¹³;
R¹-R¹³ sont, identiques ou différents, des atomes d'hydrogène, un groupe hydrocarboné qui peut éventuellement être substitué et peut également contenir des hétéroatomes, de préférence O et/ou F, avec 1 à 22 atomes de carbone ou Ar⁴, Ar⁴ identique ou différent de Ar¹ ayant les mêmes significations que Ar¹;
n est 1, 2 ou 3.

2. Polymère selon la revendication 1, caractérisé en ce qu'il est constitué de motifs de structure de formule (I).

3. Polymère selon la revendication 1 et/ou 2, caractérisé en ce qu'il présente 2 à 1000 motifs de répétition.

4. Polymère selon une ou plusieurs des revendications précédentes, caractérisé en ce qu'il est un copolymère.

5. Polymère selon la revendication 4, caractérisé en ce qu'il présente différents motifs de structure de formule (I).

6. Polymère selon une ou plusieurs des revendications précédentes, caractérisé en ce que les symboles et indices ont les significations suivantes de la formule (I) :
Ar¹, Ar² sont les groupes suivants identiques ou différents 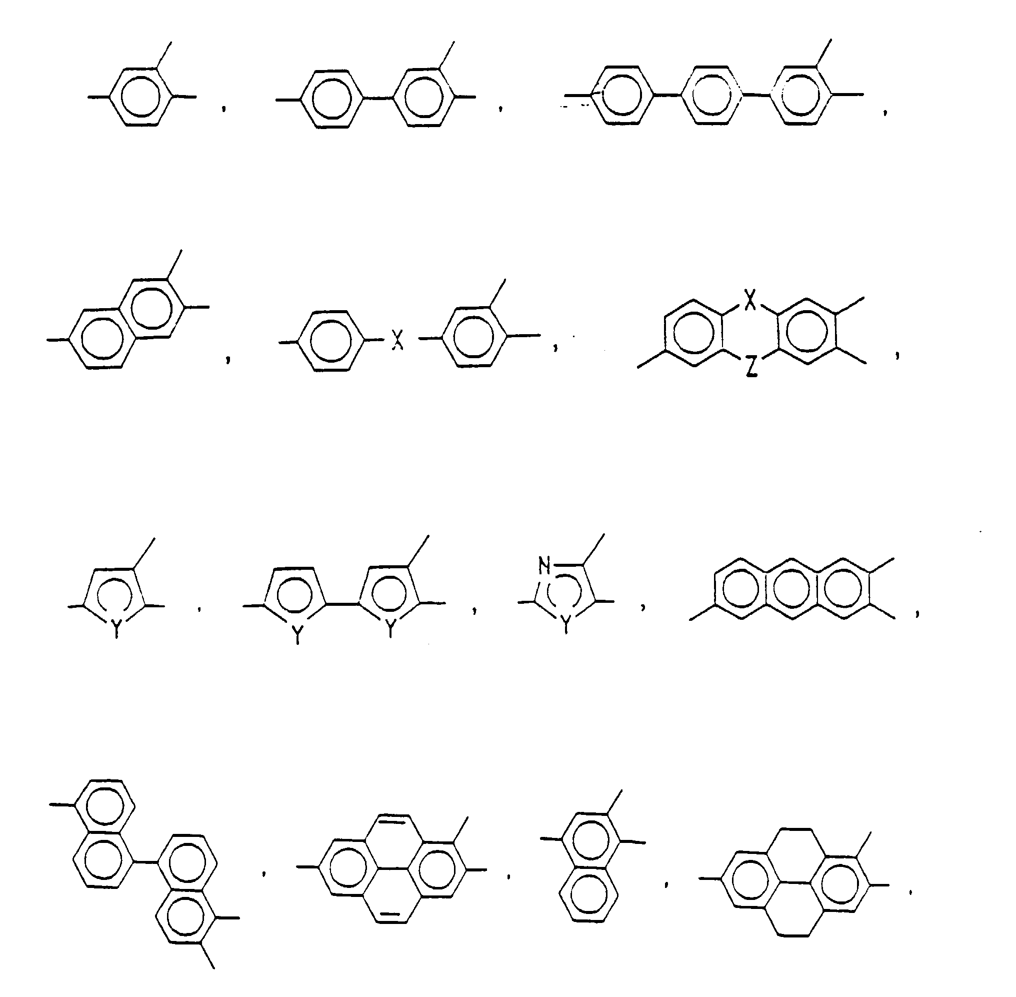 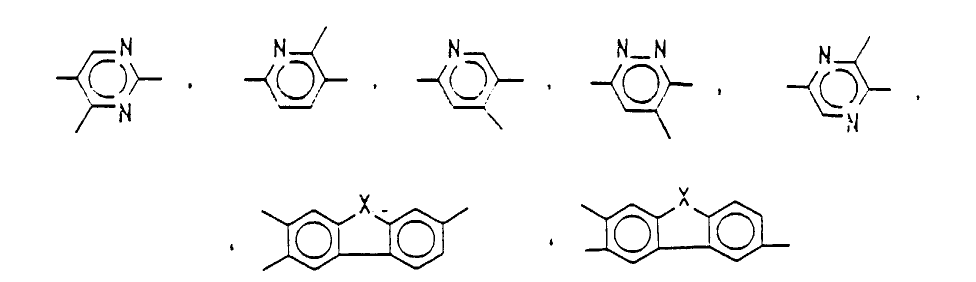
Ar³ identique ou différent de Ar¹, a les mêmes significations que Ar¹, dans lesquelles, respectivement, parmi les trois possibilités de liaison seulement deux sont réalisées ou est 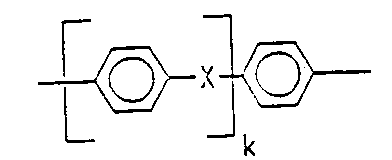 avec k = 1 à 20;
Ar¹, Ar², Ar³ peuvent, identiques ou différents, être substitués avec un ou plusieurs groupes R¹⁴;
X, Z sont, identiques ou différents, une simple liaison, -O-, -S-, -SO-, -SO₂-, NR³, -CR⁴R⁵-, -CO-, -CR⁶=CR⁷, -CR⁸R⁹-CR¹⁰R¹¹ ou SiR¹²R¹³;
Y représente -O-, -S- ou -NR³-;
R¹-R¹³ sont des atomes d'hydrogène, un groupe alkyle à chaîne linéaire ou ramifiée avec 1 à 22 atomes de carbone, dans lequel un ou deux groupes CH₂ non voisins peuvent être remplacés par -O-, -S-, -CO-, -CO-O-, -O-OC- ou -Si(CH₃)₂-, un groupe cycloalkyle avec 3 à 10 atomes de carbone ou Ar⁴, dans lequel Ar⁴, identique ou différent de Ar¹⁻³, a les mêmes significations que Ar¹⁻³, bien que parmi les trois liaisons possibles seulement une soit réalisée, R⁴-R¹¹ représentent aussi F, CF₃, O-CF₃ ou CN;
R¹⁴ représente, identiques ou différents, un atome d'hydrogène, un groupe alkyle avec 1 à 22 atomes de carbone, dans lequel également un ou deux groupes CH₂ non voisins peuvent être remplacés par -O-, -S-, -CO-, -CO-O-, -O-OC- ou -Si(CH₃)₂-, -CF₃, -Ph, -O-Ph, -S-Ph, -SO-Ph, -SO₂-Ph, F, Cl, Br, I ou -CN;
n est 1, 2 ou 3.

7. Polymère selon une ou plusieurs des revendications précédentes, caractérisé en ce que les symboles et indices ont les significations suivantes de la formule (I) :
Le groupe 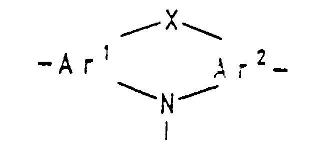 est 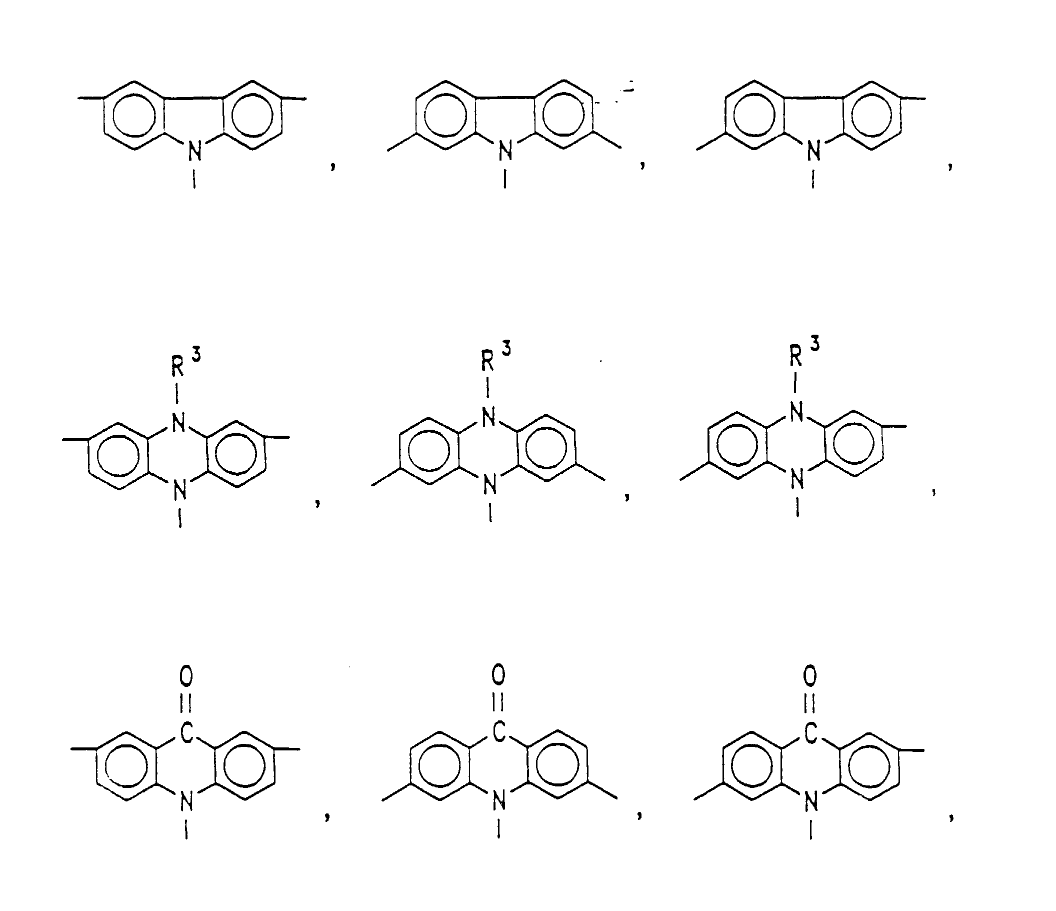 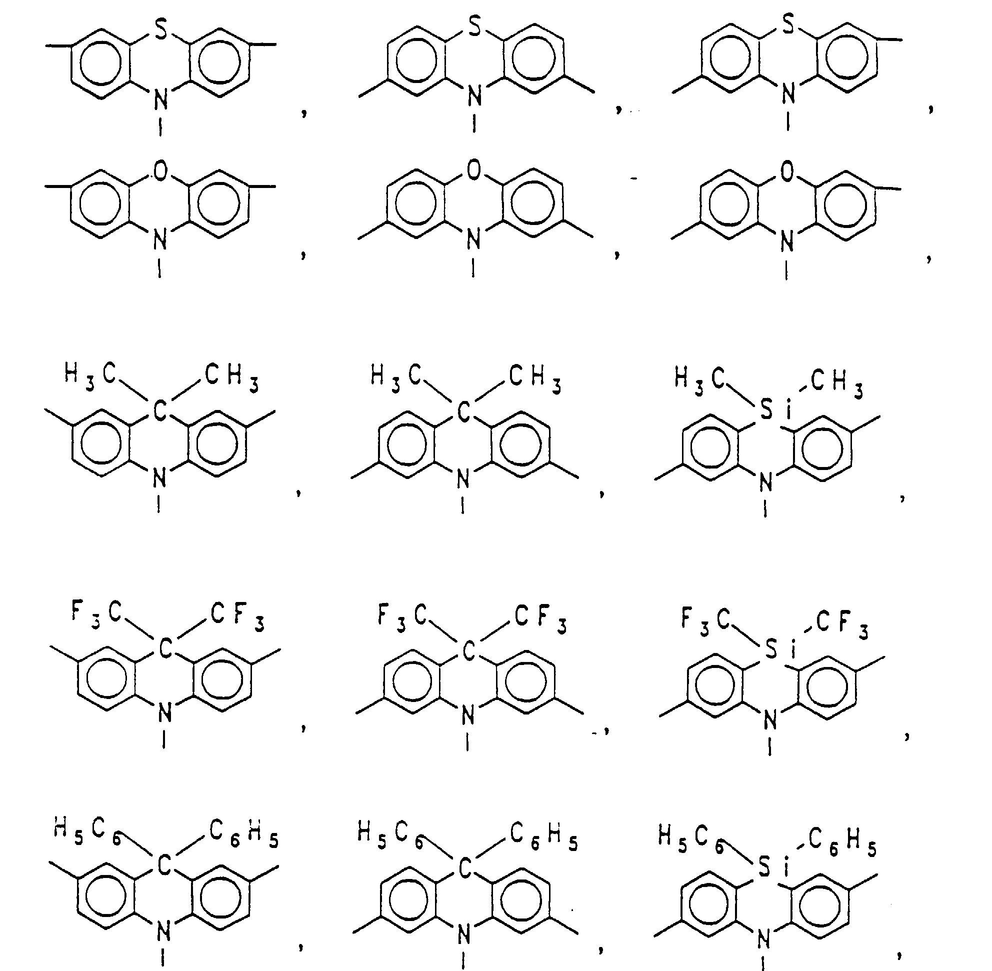
Ar³ est 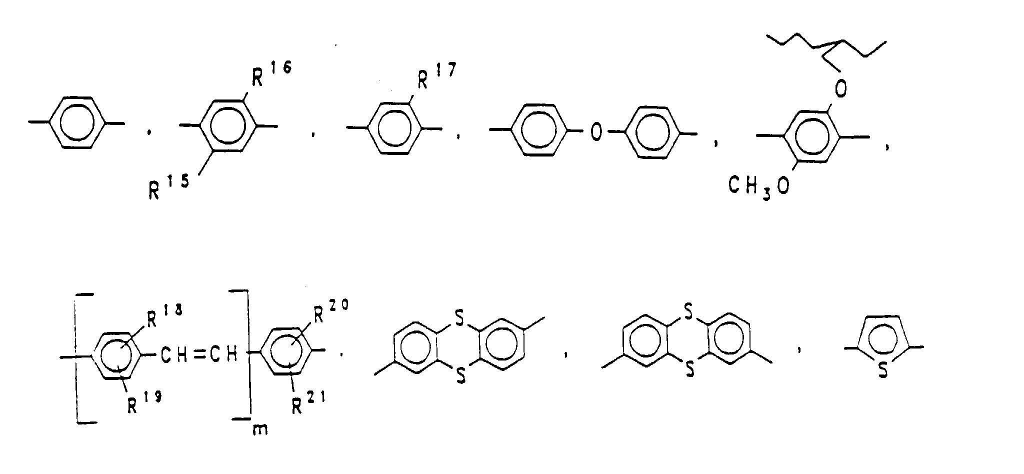 avec m = 1 ... 20, de préférence 1, 2 ou 3, 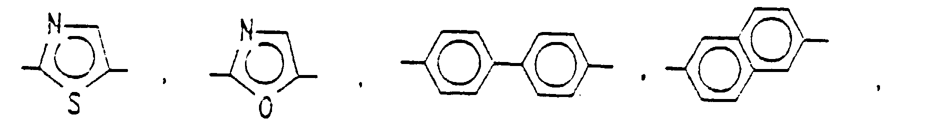
R¹, R² identiques ou différents de Ar³, ont les mêmes significations que Ar³, dans lesquelles cependant seule une des deux positions possibles de liaison au polymère est réalisée, ou sont 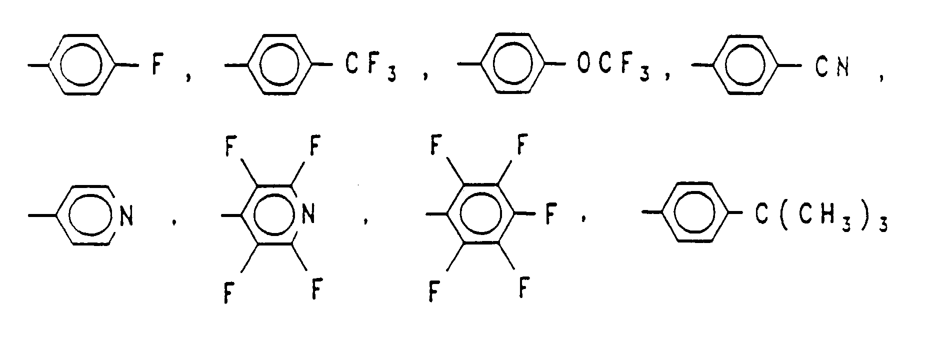 ou un atome d'hydrogène, un groupe alkyle à chaîne linéaire ou ramifiée avec 1 à 12 atomes de carbone, dans lequel également un ou deux groupes -CH₂- non voisins peuvent être remplacés par -O-, -S-, -CO-, -CO-O-, -O-OC- ou -Si(CH₃)₂-, un groupe cycloalkyle avec 3 à 6 atomes de carbone ou un groupe aryle avec 3 à 6 atomes de carbone ou un groupe aralkyle avec 7 à 11 atomes de carbone;
R¹⁵-R²¹, identiques ou différents, représentent un atome de fluor, de chlore, un groupe alkyle ou alcoxy à chaîne linéaire ou ramifiée avec 1 à 22 atomes de carbone,
R¹⁸-R²¹ peuvent aussi représenter un atome d'hydrogène.

8. Polymère selon une ou plusieurs des revendications précédentes, caractérisé en ce que Ar¹ et Ar² sont identiques.

9. Procédé de la préparation d'un polymère selon une ou plusieurs des revendications précédentes, caractérisé en ce que l'on réalise la condensation de bis-aldéhydes ou de bicétones de formule (II), 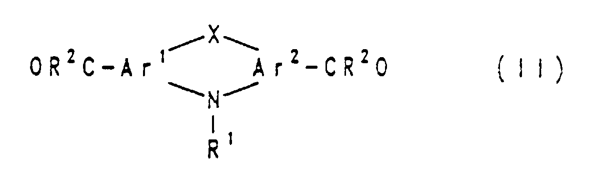 dans laquelle Ar¹, Ar², R¹, R² et X ont les significations données dans la formule (I), avec des composés organophosphorés de formule (III) 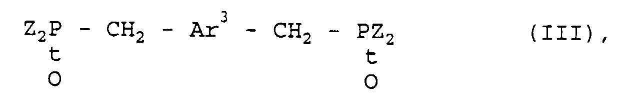 dans laquelle Ar³ possède les significations données à la formule (I) et Z représente un groupe alcoxy en C₁-C₂₂.

10. Utilisation d'un polymère selon une ou plusieurs des revendications 1 à 8 comme matériau électro-luminescent.

11. Matériau électroluminescent, contenant un ou plusieurs polymères selon une ou plusieurs des revendications 1 à 8.

12. Procédé de préparation d'un matériau électro-luminescent selon la revendication 11, caractérisé en ce que
a) l'on condense un composé organophosphoré de formule (III) 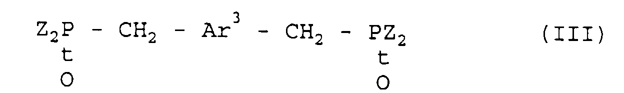 avec un bisaldéhyde ou une bicétone de formule (II) 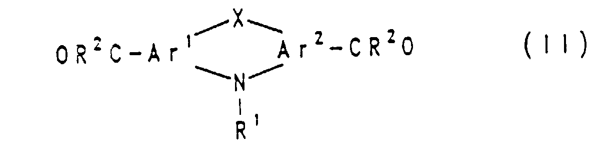 sous l'action d'un agent de condensation basique pour donner un polymère, contenant des motifs de structure de formule (I), 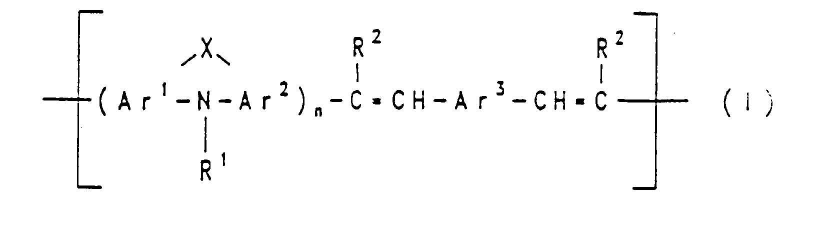 dans laquelle les symboles et indices ont les significations suivantes :
Ar¹, Ar², Ar³ sont des groupes aryle et/ou hétéroaryle identiques ou différents, avec de 4 à 400 atomes de carbone, à un et/ou plusieurs cycles, éventuellement couplés par un ou plusieurs ponts, et/ou condensés, qui peuvent être éventuellement substitués.
X est une simple liaison, -O-, -S-, -SO-, -SO₂-, NR³, -CR⁴R⁵-, -CO-, -CR⁶=CR⁷, -CR⁸R⁹-CR¹⁰R¹¹ ou SiR¹²R¹³;
R¹-R¹³ sont, identiques ou différents, des atomes d'hydrogène, un groupe hydrocarboné avec 1 à 22 atomes de carbone ou Ar⁴, dans lequel Ar⁴ peut, identiques ou différents, avoir les mêmes significations que Ar¹;
n est 1, 2 ou 3,
et
b) l'on dépose le polymère ainsi obtenu, contenant des motifs de structure de formule (I), sous la forme d'un film sur un substrat, qui contient éventuellement déjà d'autres couches.

13. Dispositif électroluminescent avec une ou plusieurs couches actives, dans lequel au moins une de ces couches actives contient un ou plusieurs polymères selon une ou plusieurs de revendications 1 à 8.
